(19) Europäisches Patentamt
European Patent Office
Office européen des brevets

(11) Veröffentlichungsnummer : **0 224 579 B1**

(12) **EUROPÄISCHE PATENTSCHRIFT**

(45) Veröffentlichungstag der Patentschrift : **24.01.90**

(51) Int. Cl.$^5$ : **C 09 K 19/34**, C 09 K 19/42

(21) Anmeldenummer : **86904119.4**

(22) Anmeldetag : **16.05.86**

(86) Internationale Anmeldenummer : **PCT/EP 86/00294**

(87) Internationale Veröffentlichungsnummer : **WO/8607085 (04.12.86 Gazette 86/26)**

# (54) SMEKTISCHE FLÜSSIGKRISTALLINE PHASEN.

(30) Priorität : **24.05.85 DE 3518734**

(43) Veröffentlichungstag der Anmeldung : **10.06.87 Patentblatt 87/24**

(45) Bekanntmachung des Hinweises auf die Patenterteilung : **24.01.90 Patentblatt 90/04**

(84) Benannte Vertragsstaaten : **CH DE FR GB LI**

(56) Entgegenhaltungen :
**EP-A- 199 004**
**EP-A-00 251 19**
**WO-A-86 /040 60**
**JP-A-56 163 171**
**JP-A-56 164 170**

(73) Patentinhaber : **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder : **SCHEUBLE, Bernhard**
**Am Grenzweg 18**
**D-6146 Alsbach (DE)**
Erfinder : **BOFINGER, Klaus**
**Eberstädter Strasse 7**
**D-6109 Mòhltal (DE)**
Erfinder : **HOPF, Reinhard**
**Wolfsgasse 3**
**D-6432 Heringen/Werra (DE)**
Erfinder : **PAUSCH, Axel**
**Rosenweg 7**
**D-6104 Seeheim (DE)**
Erfinder : **EIDENSCHINK, Rudolf**
**Kornblumenstrasse 1**
**D-6115 Mònster (DE)**
Erfinder : **KRAUSE, Joachim**
**Samuel-Morse-Strasse 14**
**D-6110 Dieburg (DE)**
Erfinder : **WAECHTLER, Andreas**
**Goethestrasse 34**
**D-6103 Griesheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Verwendung von Verbindungen der Formel I

$$R^1-A^1-Z^1-A^2-[Z^2-A^3]_m-R^2 \qquad I$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können, einer der Reste $R^1$ und $R^2$ auch H, F, Cl, Br oder CN,

$A^1$ —Pyr—Phe—, —Phe—Pyr—, —Pyr—Cy— oder —Cy—Pyr—,

Pyr Pyrimidin-2,5-diyl, Pyridin-2,5-diyl, Pyrazin-2,5-diyl oder Pyridazin-3,6-diyl,

Phe unsubstituiertes oder durch eine oder zwei F- und/oder Cl-Atome und/oder $CH_3$- und/oder CN-Gruppen substituiertes 1,4-Phenylen,

Cy trans-1,4-Cyclohexylen,

$A^2$ und $A^3$ jeweils trans-1,4-Cyclohexylen oder unsubstituiertes oder durch ein oder zwei F- und/oder Cl-Atome und/oder $CH_3$- und/oder CN-Gruppen substituiertes 1,4-Phenylen, worin auch eine oder mehrere CH-Gruppen durch N ersetzt sein können,

$Z^1$ —$CH_2CH_2$—, —CO—O—, —O—CO—, —$CH_2$—O—, oder —$OCH_2$—,

$Z^2$ —CO—O, —O—CO—, —$CH_2CH_2$—, —$CH_2O$—, —O—$CH_2$— oder eine Einfachbindung, und

m 0 oder 1

bedeuten, als Komponenten smektischer flüssigkristalliner Phasen mit ferroelektrischen Eingenschaften, smektische flüssigkristalline Phasen, insbesondere chirale getiltete smektische Phasen mit ferroelektrischen Eigenschaften, enthaltend Verbindungen der Formel I, sowie neue Verbindungen der Formeln II und III (Teilformeln der Formel I).

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L. A. Beresnev et al., Mol. Cryst. Liq. Cryst. 89, 327 (1982) ; H. R. Brand et al., J. Physique 44 (lett.), L-771 (1983). Solche Phasen können als Dielektrika für schnell schaltende Displays verwendet werden, die auf dem von Clark und Lagerwall beschriebenen Prinzip der SSFLC-Technologie (N. A. Clark und S. T. Lagerwall, Appl. Phys. Lett. 36, 899 (1980) ; USP 4, 367, 924) auf der Basis der ferroelektischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 μm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z. B. Sc*) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und der Tilt der Phasen nicht den Anforderungen der Display-Technologie entspricht. Darüberhinaus ist meist der Temperaturbereich der ferroelektrischen Phasen zu klein und liegt überwiegend bei zu hohen Temperaturen.

Es wurde nun gefunden, daß die Verwendung von Verbindungen der Formel I als Komponenten chirale getilteter smektischer Mischungen die erwähnten Nachteile wesentlich vermindern kann. Die Verbindungen der Formel I sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, insbesondere mit breiten Sc* Phasenbereichen, hervorragender Unterkühlbarkeit bis zu Temperaturen weit unter 0 °C ohne daß Kristallisation auftritt (selbst erfindungsgemäße Phasen mit einem Schmelzpunkt oberhalb 0 °C sind im allgemeinen bis weit unter 0 °C unterkühlbar), günstiger Pitchhöhe und für derartige Phasen hohen Werten für die spontane Polarisation herstellbar. P ist die spontane Polarisation in nC/$cm^2$.

Aus WO-A-86/04060, EP-A-199004, JP-A-56-164170, JP-A-56-164171 und EP-A-25119 sind ähnliche Verbindungen bekannt. Die erfindungsgemäßen Verbindungen der Formel I unterscheiden sich von den vorbekannten Verbindungen durch zusätzliche flüssigkristalline Eigenschaften, d. h., durch die Verwendbarkeit für chirale getiltete smektische flüssigkristalline Phasen mit ferroelektrischen Eigenschaften.

Die Verbindungen der Formel I besitzen einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind ; es können aber auch Verbindungen der Formel I flüssigkristallinen Basismaterialien aus anderen Verbindungklassen zugesetzt werden, um beispielsweise die dielektrische und/oder optische Anisotropie und/oder die Viskosität und/oder die spontane Polarization und/oder den Phasenbereiche und/oder den Tiltwinkel und/oder den Pitch eines solchen Dielektrikums zu variieren.

Gegenstand der Erfindung ist somit die Verwendung der Verbindungen der Formel I als Komponenten chirale getilteter smektischer flüssigkristalliner Phasen mit ferroelektrischen Eigenschaften. Gegenstand der Erfindung sind ferner smektische flüssigkristalline Phasen, insbesondere chirale getiltete smektische Phasen mit ferroelektrischen Eigenschaften, mit einem Gehalt an mindestens einer Verbindung der Formel I sowie Flüssigkristallanzeigeelemente, insbesondere elektrooptische Anzeigeelemente, die derartige Phasen enthalten.

Der Einfachheit halber bedeuten im folgenden Cy eine 1,4-Cyclohexylengruppe und Phe eine 1,4-Phenylengruppe, die gegebenenfalls auch durch ein oder zwei F- und/oder Cl-Atome und/oder $CH_3$- und/oder CN-Gruppen substituiert sein kann (= Phe(F), Phe(Cl), Phe($CH_3$), Phe(CN)).

Die Verbindungen der Formel I umfassen insbesondere Verbindungen der Teilformeln Ia, Ib, Ie und If (mit drei Ringen), sowie Ic, Id, Ig, Ih, Ii und Ij (mit vier Ringen) :

$R^1$—Pyr—Phe—$Z^1$—Phe—$R^2$ Ia
$R^1$—Pyr—Cy—$Z^1$—Phe—$R^2$ Ib
$R^1$—Pyr—Phe—$Z^1$—Phe—$Z^2$—$A^3$—$R^2$ Ic
$R^1$—Pyr—Cy—$Z^1$—Phe—$Z^2$—$A^3$—$R^2$ Id
$R^1$—Phe—Pyr—$Z^1$—Phe—$R^2$ Ie
$R^1$—Cy—Pyr—$Z^1$—Phe—$R^2$ If
$R^1$—Phe—Pyr—$Z^1$—Phe—$Z^2$—$A^3$—$R^2$ Ig
$R^1$—Cy—Pyr—$Z^1$—Phe—$Z^2$—$A^3$—$R^2$ Ih
$R^1$—Phe—Pyr—$Z^1$—$A^2$—$Z^2$—Phe—$R^2$ Ii
$R^1$—Pyr—Phe—$Z^1$—$A^2$—$Z^2$—Phe—$R^2$ Ij

Darunter sind diejenigen der Teilformeln Ia, Ic und Ie bevorzugt.

Die bevorzugten Verbindungen der Teilformel Ia umfassen solche der Teilformeln Iaa bis Iae :

$R^1$—Pyr—Phe—$OCH_2$—Phe—$R^2$ Iaa
$R^1$—Pyr—Phe—$CH_2CH_2$—Phe—$R^2$ Iab
$R^1$—Pyr—Phe—$CH_2O$—Phe—$R^2$ Iac
$R^1$—Pyr—Phe—OCO—Phe—$R^2$ Iad
$R^1$—Pyr—Phe—COO—Phe—$R^2$ Iae

Darunter sind diejenigen der Teilformeln Iaa und Iad besonders bevorzugt. Ferner bevorzugt ist Iae.

Die bevorzugten Verbindungen der Teilformel Ib umfassen solche der Teilformeln Iba bis Ibc :

$R^1$—Pyr—Cy—$OCH_2$—Phe—$R^2$ Iba
$R^1$—Pyr—Cy—$CH_2CH_2$—Phe—$R^2$ Ibb
$R^1$—Pyr—Cy—$CH_2O$—Phe—$R^2$ Ibc

Bevorzugte Verbindungen der Teilformel Ic sind diejenigen der Teilformeln Ica bis Icg :

$R^1$—Pyr—Phe—$OCH_2$—Phe—OCO—Cy—$R^2$ Ica
$R^1$—Pyr—Phe—$OCH_2$—Phe—OCO—Phe—$R^2$ Icb
$R^1$—Pyr—Phe—$OCH_2$—Phe—Pyr—$R^2$ Icc
$R^1$—Pyr—Phe—OCO—Phe—Phe—$R^2$ Icd
$R^1$—Pyr—Phe—OCO—Phe—Pyr—$R^2$ Ice
$R^1$—Pyr—Phe—COO—Phe—Phe—$R^2$ Icf
$R^1$—Pyr—Phe—COO—Pyr—Phe—$R^2$ Icg

Darunter sind diejenigen der Teilformeln Ica, Icd, Ice, Icf und Icg, insbesondere Ice, besonders bevorzugt.

Die bevorzugten Verbindungen der Teilformel Ie umfassen solche der Teilformeln Iea bis Iee :

$R^1$—Phe—Pyr—$OCH_2$—Phe—$R^2$ Iea
$R^1$—Phe—Pyr—$CH_2CH_2$—Phe—$R^2$ Ieb
$R^1$—Phe—Pyr—$CH_2O$—Phe—$R^2$ Iec
$R^1$—Phe—Pyr—OCO—Phe—$R^2$ Ied

$R^1$—Phe—Pyr—COO—Phe—$R^2$ Iee

Darunter sind diejenigen der Teilformel Iea, Ied und Iee besonders bevorzugt.

Die bevorzugten Verbindungen der Teilformel If umfassen solche der Teilformeln Ifa bis Ife :

$R^1$—Cy—Pyr—$OCH_2$—Phe—$R^2$ Ifa
$R^1$—Cy—Pyr—$CH_2CH_2$—Phe—$R^2$ Ifb
$R^1$—Cy—Pyr—$CH_2O$—Phe—$R^2$ Ifc
$R^1$—Cy—Pyr—OCO—Phe—$R^2$ Ifd
$R^1$—Cy—Pyr—COO—Phe—$R^2$ Ife

Die bevorzugten Verbindungen der Teilformel Ig umfassen solche der Teilformeln Iga bis Igk :

$R^1$—Phe—Pyr—$OCH_2$—Phe—Cy—$R^2$ Iga
$R^1$—Phe—Pyr—OCO—Phe—OCO—Phe—$R^2$ Igb
$R^1$—Phe—Pyr—OCO—Phe—OCO—Cy—$R^2$ Igc
$R^1$—Phe—Pyr—OCO—Phe—COO—Phe—$R^2$ Igd
$R^1$—Phe—Pyr—OCO—Phe—COO—Cy—$R^2$ Ige
$R^1$—Phe—Pyr—COO—Phe—COO—Phe—$R^2$ Igf
$R^1$—Phe—Pyr—COO—Phe—COO—Cy—$R^2$ Igg
$R^1$—Phe—Pyr—COO—Phe—OCO—Phe—$R^2$ Igh
$R^1$—Phe—Pyr—COO—Phe—OCO—Cy—$R^2$ Igi
$R^1$—Phe—Pyr—COO—Phe—Pyr—$R^2$ Igj
$R^1$—Phe—Pyr—OCO—Phe—Pyr—$R^2$ Igk

Darunter sind diejenigen der Teilformel Igk besonders bevorzugt.

Die bevorzugten Verbindungen der Teilformel Ii umfassen solche der Teilformeln Iia bis Iid :

$R^1$—Phe—Pyr—$OCH_2$—Phe—Phe—$R^2$ Iia
$R^1$—Phe—Pyr—$OCH_2$—Phe—$CH_2O$—Phe—$R^2$ Iib
$R^1$—Phe—Pyr—$CH_2O$—Phe—Phe—$R^2$ Iic
$R^1$—Phe—Pyr—OCO—Phe—Phe—$R^2$ Iid

Die bevorzugten Verbindungen der Teilformel Ij umfassen solche der Teilformeln Ija bis Ijd :

$R^1$—Pyr—Phe—$OCH_2$—Phe—Phe—$R^2$ Ija
$R^1$—Pyr—Phe—OCO—Phe—Phe—$R^2$ Ijb
$R^1$—Pyr—Phe—COO—Pyr—Phe—$R^2$ Ijc
$R^1$—Pyr—Phe—COO—Phe—Phe—$R^2$ Ijd

In den Verbindungen der vor- und nachstehenden Formeln bedeuten $R^1$ und $R^2$ vorzugsweise Alkyl oder Alkoxy, ferner Oxaalkyl, Alkanoyloxy oder Alkoxycarbonyl.

$A^1$ ist bevorzugt —Pyr—Phe— oder Phe—Pyr—, insbesondere bevorzugt —Pyr—Phe—, —$A^1$— ist weiterhin vorzugsweise ein Strukturelement ausgewählt aus der gruppe der Formeln 1 bis 4 :

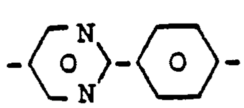,
1

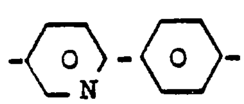,
2

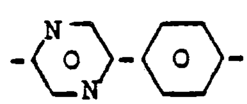,
3

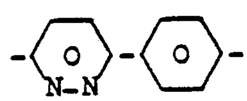
4

Gruppen der Formeln 1 und 2 sind besonders bevorzugt.

$A^2$ ist bevorzugt unsubstituiertes 1,4-Phenylen oder trans-1,4-Cyclohexylen.

$Z^1$ ist vorzugsweise —$OCH_2$—, —O—CO— oder —CO—O.

m ist vorzugsweise 0.

$Z^2$ ist vorzugsweise eine Einfachbindung, —CO—O— oder —O—CO—, insbesondere bevorzugt —O—CO—.

$A^3$ ist vorzugsweise Cy oder Phe.

Die Alkylreste in den Gruppen $R^1$ und/oder $R^2$ können geradkettig oder verzweigt sein. Vorzugsweise sind sie geradkettig, haben 5, 6, 7, 8, 9, 10, 11 oder 12 C-Atome und bedeuten demnach bevorzugt Pentyl, Hexyl, Heptyl, Octyl, Nonyl, Decyl, Undecyl oder Dodecyl, ferner Methyl, Ethyl, Propyl, Butyl.

Falls $R^1$ und/oder $R^2$ Alkylreste bedeuten, in denen eine (« Alkoxy » bzw. « Oxaalkyl ») oder zwei (« Alkoxyalkoxy » bzw. « Dioxaalkyl » $CH_2$-Gruppen durch O-Atome ersetzt sind, so können sie geradkettig oder verzweigt sein. Vorzugsweise sind sie geradkettig, haben 5, 6, 7, 8, 9, 10, 11 oder 12 C-Atome und bedeuten demnach bevorzugt Pentoxy, Hexoxy, Heptoxy, Octoxy, Nonoxy, Decoxy, Undecoxy, Dodecoxy,

2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3- 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl, 1,4-Dioxaoctyl, 1,4,7-Trioxaoctyl, 1,4-Dioxanonyl, 1,4-Dioxadecyl.

Alkenylgruppen in den Verbindungen der Formel I sind vorzugsweise geradkettige trans-Alkenylgruppen der Formel

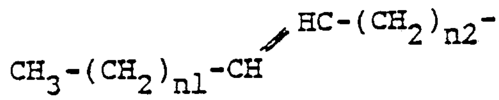

worin n2 0 bis 10, vorzugsweise 2 bis 10, und n1 0 bis 5, vorzugsweise 0, bedeutet.

Verbindungen der Formel I sowie der vor- und nachstehenden Teilformeln mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe für chiral getiltete smektische Phasen, wenn sie optisch aktiv sind. Solche Verbindungen eignen sich jedoch auch als Komponenten nematischer flüssigkristalliner Phasen, insbesondere zur Vermeidung von reverse twist. Verzweigte Gruppen dieser Art enthalten in der Regel nicht mehr als eine Kettenverzweigung. Bevorzugte verzweigte Reste sind Isopropyl, 2-Butyl (= 1-Methylpropyl), Isobutyl (= 2-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-octyloxy.

Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, Cl oder Br), Alkyl oder Alkoxy mit jeweils 1 bis 5 C-Atomen und CN verknüpft. Der optisch aktive organische Rest hat vorzugsweise die Formel,

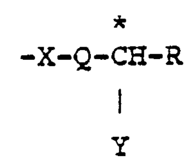

worin

X —CO—O—, —O—CO—, —O—CO—O—, —CO—, —O—, —S—, —CH=CH—, —CH=CH—COO— oder eine Einfachbindung,

Q Alkylen mit 1 bis 5 C-Atomen, worin auch eine nicht mit X verknüpfte $CH_2$-Gruppe durch —O—, —CO—, —O—CO—, —CO—O— oder CH=CH— ersetzt sein kann, oder eine Einfachbindung,

Y CN, Halogen, Methyl oder Methoxy, und

R eine von Y verschiedene Alkylgruppe mit 1 bis 18 C-Atomen, worin auch eine oder zwei nicht benachbarte $CH_2$-Gruppen durch —O—, —CO—, —O—CO—, —CO—O und/oder —CH=CH— ersetzt sein können,

bedeutet.

X ist vorzugsweise —CO—O—, —O—CO—, —O—, —CH=CH—COO— (trans) oder eine Einfachbindung. Besonders bevorzugt sind —O—, —CO—O— und —O—CO—.

Q ist vorzugsweise Alkylen mit 1 bis 5 C-Atomen oder eine Einfachbindung, insbesondere bevorzugt —$CH_2$—, —$CH_2CH_2$ oder eine Einfachbindung.

Y ist vorzugsweise $CH_3$, —CN oder Cl, insbesondere bevorzugt —CN oder Cl.

R ist vorzugsweise geradkettiges Alkyl mit 1 bis 10, insbesondere mit 1 bis 7, C-Atomen, worin gegebenenfalls die mit dem asymmetrischen C-Atom verknüpfte $CH_2$-Gruppe durch —O—, —O—CO— oder —CO—O— ersetzt sein kann.

Vorzugsweise ist $R^2$ in Formel I der optisch aktive Rest.

Besonders bevorzugte optisch aktive Reste entsprechen hier der Formel

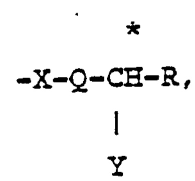

worin X

—O—, —CO—O— oder —O—CO—, Q —$CH_2$— oder eine Einfachbindung, Y $CH_3$ und R geradkettiges Alkyl mit 1 bis 7 C-Atomen bedeutet, worin die mit den asymmetrischen C-Atomen verknüpfte $CH_2$-Gruppe durch —O—, —CO—O— oder —O—CO— ersetzt ist.

Unter den verbindungen der Formel I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I56:

| | |
|---|---|
| R—Pyr—Phe—$OCH_2$—Phe—R′ | I1 |
| R—Pyr—Phe—$CH_2O$—Phe—R′ | I2 |
| R—Pyr—Phe—OCO—Phe—R′ | I3 |
| R—Pyr—Phe—COO—Phe—R′ | I4 |
| R—Pyr—Phe—$CH_2CH_2$—Phe—R′ | I5 |
| R—Phe—Pyr—$OCH_2$—Phe—R′ | I6 |
| R—Phe—Pyr—$CH_2O$—Phe—R′ | I7 |
| R—Phe—Pyr—OCO—Phe—R′ | I8 |
| R—Phe—Pyr—COO—Phe—R′ | I9 |
| R—Phe—Pyr—$CH_2CH_2$—Phe—R′ | I10 |
| R—Pyr—Phe—$OCH_2$—Cy—R′ | I11 |
| R—Pyr—Phe—$CH_2O$—Cy—R′ | I12 |
| R—Pyr—Phe—OCO—Cy—R′ | I13 |
| R—Pyr—Phe—COO—Cy—R′ | I14 |
| R—Pyr—Phe—$CH_2CH_2$—Cy—R′ | I15 |
| R—Phe—Pyr—$OCH_2$—Cy—R′ | I16 |
| R—Phe—Pyr—$CH_2O$—Cy—R′ | I17 |
| R—Phe—Pyr—OCO—Cy—R′ | I18 |
| R—Phe—Pyr—COO—Cy—R′ | I19 |
| R—Phe—Pyr—$CH_2CH_2$—Cy—R′ | I20 |
| R—Pyr—Phe—$OCH_2$—Phe—OCO—Cy—R′ | I21 |
| R—Pyr—Phe—$OCH_2$—Phe—OCO—Phe—R′ | I22 |
| R—Phe—Pyr—$OCH_2$—Phe—OCO—Cy—R′ | I23 |
| R—Phe—Pyr—$OCH_2$—Phe—OCO—Phe—R′ | I24 |
| R—Pyr—Phe—OCO—Phe—OCO—Cy—R′ | I25 |
| R—Pyr—Phe—COO—Phe—OCO—Cy—R′ | I26 |
| R—Phe—Pyr—OCO—Phe—OCO—Cy—R′ | I27 |
| R—Phe—Pyr—COO—Phe—OCO—Cy—R′ | I28 |
| R—Pyr—Phe—OCO—Phe—OCO—Phe—R′ | I29 |
| R—Pyr—Phe—COO—Phe—OCO—Phe—R′ | I30 |
| R—Pyr—Phe—OCO—Phe—OCO—Phe—R′ | I31 |
| R—Phe—Pyr—COO—Phe—OCO—Phe—R′ | I32 |
| R—Pyr—Phe—$OCH_2$—Phe—Cy—R′ | I33 |
| R—Pyr—Phe—$OCH_2$—Phe—Phe—R′ | I34 |
| R—Pyr—Phe—$OCH_2$—Phe—Pyr—R′ | I35 |
| R—Pyr—Phe—OCO—Phe—Cy—R′ | I36 |
| R—Pyr—Phe—OCO—Phe—Phe—R′ | I37 |
| R—Pyr—Phe—OCO—Phe—Pyr—R′ | I38 |
| R—Phe—Pyr—$OCH_2$—Phe—Cy—R′ | I39 |
| R—Phe—Pyr—$OCH_2$—Phe—Phe—R′ | I40 |
| R—Phe—Pyr—$OCH_2$—Phe—Pyr—R′ | I41 |
| R—Phe—Pyr—OCO—Phe—Cy—R′ | I42 |
| R—Phe—Pyr—OCO—Phe—Phe—R′ | I43 |
| R—Phe—Pyr—OCO—Phe—Pyr—R′ | I44 |
| R—Phe—Pyr—$OCH_2$—Pyr—Phe—R′ | I45 |
| R—Phe—Pyr—OCO—Pyr—Phe—R′ | I46 |
| R—Pyr—Phe—OCO—Phe(F)—R′ | I47 |
| R—Pyr—Phe—COO—Phe(F)—R′ | I48 |
| R—Phe—Pyr—OCO—Phe(F)—R′ | I49 |
| R—Pyr—Phe—$CH_2O$—Phe(F)—R′ | I50 |
| R—Pyr—Phe(F)—OCO—Phe—R′ | I51 |
| R—Pyr—Phe(F)—OCO—Cy—R′ | I52 |

R—Pyr—Phe(F)—COO—Phe—R' I53
R—Pyr—Phe(F)—COO—Cy—R' I54
R—Pyr—Phe—COO—Phe(2—CN)—R' I55
R—Pyr—Phe—COO—Phe(2—CN, 3—CN)—R' I56

In den vorstehenden Formeln I1 bis I56 bedeuten R und R' vorzugsweise geradkettige Alkyl-, Alkoxy-, Alkanoyloxyoder Alkoxycarbonylgruppen mit jeweils 5 bis 12 C-Atomen.

Die vorstehend gennanten Formeln umschließen die beiden möglichen 2,5-Stellungsisomeren bezüglich der Pyridin-2,5-bzw. Pyrimidin-2,5-diyl-Gruppe. Vorzugsweise ist diese Gruppe in 5-Position mit $R^1$ verknüpft.

Verbindungen der Formel I, die keine $S_c$-Phasen aufweisen, eignen sich ebenfalls als Komponenten erfindungsgemäßer smektischer Phasen.

Alle Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen (z. B. DE-OS 23 44 732, 24 50 088, 24 29 093, 25 02 904, 26 36 684, 27 01 591 und 27 52 975 betreffend Verbindungen mit 1,4-Cyclohexylen und 1,4-Phenylen-Gruppen ; DE-PS 26 41 724 betreffend Verbindungen mit Pyrimidin-2,5-diyl-Gruppen ; DE-OS 29 44 905 und 32 27 916 betreffend Verbindungen mit 1,3-Dioxan-2,5-diyl-Gruppen) ; DD 160 061 betreffend Verbindungen mit 1,3-Dithian-2,5-diyl-Gruppen ; US 4, 261, 652 und 4, 219, 256 betreffend Verbindungen mit 1,4-Bicyclo(2,2,2)-octylen-Gruppen ; und DE-OS 32 01 721 betreffend Verbindungen mit $—CH_2CH_2—$ Brückengliedern.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Im allgemeinen werden zwei entsprechende Teilverbindungen (z. B. (1) und (2) (Schema 1) oder (3) und (4) (Schema 2) zu Verbindungen der Formel I kondensiert :

Schema 1 :

$$R'\text{-}M^1L^1 \quad + \quad L^2M^2\text{-}R''$$

(1) (2)

$$\downarrow \; -L^1L^2$$

$$R'\text{-}M^1M^1\text{-}R'' \quad I \qquad (M^1M^2 = A)$$

Schema 2 :

$$R'\text{-}M^3L^3 \quad + \quad L^4M^4\text{-}R''$$

(3) (4)

$$\downarrow \; -L^3L^4$$

$$R'\text{-}M^3M^4\text{-}R'' \quad I$$

$$(M^3M^4 = Z)$$

$—M^1L^1$ und $—M^2L^2$ sind kondensationsfähige Bausteine, die z. B. Malonsäurederivaten (z. B.

Malondialdehyd), Amidinen, Aldehyden, 1,3-Propandiolen und/oder 1,3-Propandithiolen entsprechen. $L^1L^2$ sind eine oder mehrere abgespaltene Gruppen.

— $M^3L^3$ und — $M^4L^4$ sind kondensationsfähige Bausteine z. B. ausgewählt aus der Gruppe —COOH, —COHalogen, OH, —OMetall, —$CH_2$-Halogen, —$CH_2$-Metall, —$CH_2$—OH, —$CH_2$—O-Metall, -Metall, -Halogen.

$L^3$ und $L^4$ sind Abgangsgruppen. $L^3L^4$ ist eine abgespaltene Gruppe wie z. B. $H_2O$, H-Halogen, Metall-Halogen.

Weiterhin können jedoch auch entsprechende intramolekulare Kondensationen zur Synthese von Verbindungen der Formel I durchgeführt werden (z. B. Kondensation von 1,4-Diketonen mit Hydrazin (z. B. DE-OS 32 38 350) oder Umsetzung eines Butadienderivates z. B. mit Acetylendicarbonsäurederivaten (z. B. JP-OS 58-144327 ; JP-OS 58-146543).

Die Ausgangsstoffe sind bekannt oder können nach analogen Methoden wie die bekannten Verbindungen erhalten werden.

Der Fachmann kann durch Routinemethoden entsprechende Ausgangsstoffe und/oder Methoden zu deren Synthese aus dem Stand der Technik entnehmen.

Formel I umfaßt zum überwiegenden Teil bekannte Verbindungen, wie beispielsweise die bevorzugten, in den Deutschen Patentanmeldungen P 34 01 321, P 34 04 116, P 35 00 909 und P 35 06 446, in der DE-OS 33 15 295, in der EP-OS 84 194 und im USP 4, 311, 610 (EP-PS 25 119) beschriebenen Pyrimidinderivate.

Die vorliegende Erfindung betrifft jedoch ebenfalls neue Pyrimidinderivate der Formel II

$$R^1\text{-}A^1\text{-Pyr-}Z^1\text{-}A^2\text{-}[Z^2\text{-}A^3]_m\text{-}R^2 \qquad \text{II}$$

worin

$R^1$ und $R^2$ jeweils eine Alkylgruppe mit 1 bis 12 C-Atomen, worin auch eine oder zwei nicht benachbarte $CH_2$-Gruppen durch —O—, —CO—, —O—CO—, —CO—O— oder —CH=CH— ersetzt sein können, einer der Reste $R^1$ und $R^2$ auch H, F, Cl, Br oder CN,

Pyr Pyrimidin-2,5-diyl,

$A^2$ und $A^3$ jeweils trans-1,4-Cyclohexylen oder unsubstituiertes oder durch ein oder zwei F- und/oder Cl-Atome und/oder $CH_3$- und/oder CN-Gruppen substituiertes 1,4-Phenylen,

$A^1$ unsubstituiertes oder durch ein oder zwei F- und/oder Cl-Atome und/oder $CH_3$- und/oder CN-Gruppen substituiertes 1,4-Phenylen,

$Z^1$ —$OCH_2$— oder —O—CO—,

$Z^2$ —CO—O—, —O—CO—, —$CH_2CH_2$—, $CH_2O$—, —O—$CH_2$ oder eine Einfachbindung und

m 0 oder 1

bedeuten, mit der Maßgabe, daß im Falle Z' = —O—CO—, A' unsubstituiertes 1,4-Phenylen und $A^2$ und $A^3$ jeweils trans-1,4-Cyclohexylen oder unsubstituiertes 1,4-Phenylen bedeuten, sowie die Herstellung der Verbindungen der Formel II, flüssigkristalline Phasen enthaltend Verbindungen der Formel II und deren Verwendung als Komponenten flüssigkristalliner Phasen.

Die Verbindungen der Formel II besitzen eine hohe chemische Stabilität. Sie sind farblos und gut mischbar mit allen gebräuchlichen Flüssigkristallen. Ihr Einsatz in flüssigkristallinen Phasen führt zu breiteren Mesophasenbereichen und verbesserten Werten für die Spontanpolarisation in chiral getilteten smektischen Phasen. Die erfindungsgemäßen Phasen eignen sich somit sehr gut für flüssigkristalline Phasen für Displays, die auf dem Prinzip der SSFLC-Technologie beruhen. Ferner eigenen sie sich jedoch auch für andere elektrooptische Anzeigevorrichtungen, wie beispielsweise TN-Zellen oder Guest-Host-Zellen. Hier dienen sie neben der Erweiterung des Mesophasenbereichs insbesondere zur Vermeidung von reverse twist und zur Verbesserung der elastischen Konstanten.

$R^1$ und $R^2$ sind vorzugsweise Alkyl- oder Alkoxygruppen mit jeweils 3 bis 10 C-Atomen. Sie sind vorzugsweise geradkettig. Ferner bevorzugt sind jedoch Verbindungen der Formel II, worin eine der Gruppen $R^1$ und $R^2$ eine verzweigte Alkyl-oder Alkoxygruppe bedeutet.

$A^2$ und $A^3$ bedeuten vorzugsweise trans-1,4-Cyclohexylen oder unsubstituiertes 1,4-Phenylen. $A^1$ ist vorzugsweise unsubstituiertes 1,4-Phenylen. m ist vorzugsweise 0.

Formel II umfaßt besonders bevorzugte Verbindungen der Teilformeln IIa bis IIf :

| | |
|---|---|
| $R^1$—Phe—Pyr—$OCH_2$—Phe—$R^2$ | IIa |
| $R^1$—Phe—Pyr—OCO—Phe—$R^2$ | IIb |
| $R^1$—Phe—Pyr—$OCH_2$—Cy—$R^2$ | IIc |
| $R^1$—Phe—Pyr—OCO—Cy—$R^2$ | IId |
| $R^1$—Phe—Pyr—OCO—Phe—OCO—Phe—$R^2$ | IIe |
| $R^1$—Phe—Pyr—OCO—Phe—COO—Phe—$R^2$ | IIf |

Darunter sind diejenigen der Teilformeln IIa, IIb, IIc und IId besonders bevorzugt. Insbesondere

bevorzugt sind Verbindungen der Teilformeln IIa und IIb.

Die Verbindungen der Formel II können beispielsweise durch Veresterung einer entsprechenden Carbonsäure oder eines ihrer reaktionsfähigen Derivate mit einem entsprechenden Alkohol oder einem seiner reaktionsfähigen Derivate, durch Veretherung einer entsprechenden Hydroxyverbindung oder durch Umsetzung eines entsprechenden Amidins oder eines seiner Derivate mit einem 2-Acetyl-alkanaldialkylacetal oder einem seiner reaktionsfähigen Derivate hergestellt werden.

Die vorliegende Erfindung betrifft ferner neue vierkernige Verbindungen der Formel III,

$$R^1\text{-}A^1\text{-}Z^3\text{-}A^{1'}\text{-}R^2 \qquad \text{III}$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können,

$A^1$ und $A^{1'}$ jeweils —Pyr—Phe—, —Phe—Pyr—, —Pyr—Cy— oder —Cy—Pyr—,

Pyr Pyrimidin-2,5-diyl,

Phe unsubstituiertes oder durch ein oder zwei F- und/oder Cl-Atome und/oder $CH_3$- und/oder CN-Gruppen substituiertes 1,4-Phenylen,

Cy trans-1,4-Cyclohexylen, und

$Z^3$ —CO—O— oder —$CH_2$—O—

bedeutet, sowie die Herstellung der Verbindungen der Formel III, flüssigkristalline Phasen enthaltend Verbindungen der Formel III und deren Verwendung als Komponenten flüssigkristalliner Phasen.

Die Verbindungen der Formel III besitzen eine hohe chemische Stabilität. Sie sind farblos und gut mischbar mit allen gebraüchlichen Flüssigkristallen. Ihr Einsatz in flüssigkristallinen Phasen führt zu breiteren Mesophasenbereichen und verbesserten Werten für die Spontanpolarisation in chiral getilteten smektischen Phasen. Die erfindungsgemäßen Phasen eignen sich somit sehr gut für flüssigkristalline Phasen für Displays, die auf dem Prinzip der SSFLC-Technologie beruhen. Ferner eignen sie sich jedoch auch für andere elektrooptische Anzeigevorrichtungen, wie beispielsweise TN-Zellen oder Guest-Host-Zellen. Hier dienen sie neben der Erweiterung des Mesophasenbereichs insbesondere zur Vermeidung von reverse twist und zur Verbesserung der elastischen Konstanten.

$R^1$ und $R^2$ sind vorzugsweise Alkyl- oder Alkoxygruppen mit jeweils 3 bis 10 C-Atomen. Sie sind vorzugsweise geradkettig. Ferner bevorzugt sind jedoch Verbindungen der Formel III, worin eine der Gruppen $R^1$ und $R^2$ eine verzweigte Alkyl- oder Alkoxygruppe bedeutet, $A^1$ und $A^{1'}$ bedeuten vorzugsweise —Pyr—Phe— oder —Phe—Pyr—.

Formel III umfaßt besonders bevorzugte Verbindungen der Teilformeln IIIa bis IIId :

| | |
|---|---|
| $R^1$—Pyr—Phe—COO—Phe—Pyr—$R^2$ | IIIa |
| $R^1$—Pyr—Phe—COO—Pyr—Phe—$R^2$ | IIIb |
| $R^1$—Pyr—Phe—$CH_2O$—Phe—Pyr—$R^2$ | IIIc |
| $R^1$—Pyr—Phe—$CH_2O$—Pyr—Phe—$R^2$ | IIId |

Die Verbindungen der Formel III können beispielsweise durch Veresterung einer entsprechenden Carbonsäure oder ihrer reaktionsfähigen Derivate mit einem entsprechenden Alkohol oder einem seiner reaktionsfähigen Derivate, durch Veretherung einer entsprechenden Hydroxyverbindung oder durch Umsetzung eines entsprechenden Amidins oder eines seiner Derivate mit einem 2-Acetyl-alkanaldialkylacetal oder einem seiner reaktionsfähigen Derivate hergestellt werden.

Bevorzugte Komponenten der erfindungsgemäßen Phasen sind diejenigen der Formel IV

$$R^1\text{-}A^1\text{-}Z^1\text{-}A^2\text{-}[Z^2\text{-}A^3]_m\text{-}R^2 \qquad \text{IV}$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können, einer der Reste $R^1$ und $R^2$ auch H, F, Cl, Br oder CN,

$A^1$ unsubstituiertes oder durch eine oder zwei F- und/oder Cl- und/oder Br-Atome und/oder $CH_3$- und/oder CN-Gruppen substituiertes 4,4'-Biphenylyl, worin eine oder mehrere CH-Gruppen durch N ersetzt sind,

$A^2$ und $A^3$ jeweils trans-1,4-Cyclohexylen oder unsubstituiertes oder durch ein oder zwei F- und/oder Cl-Atome und/oder Br-Atome und/oder $CH_3$- und/oder CN-Gruppen substituiertes 1,4-Phenylen, worin auch eine oder mehrere CH-Gruppen durch N ersetzt sein können,

$Z^1$ —$CH_2CH_2$—, —CO—O—, —O—CO—, —$CH_2O$—, oder —$OCH_2$—,

$Z^2$ —CO—O—, —O—CO—, —$CH_2CH_2$—, —$CH_2O$—, —O—$CH_2$— oder eine Einfachbindung, und

m 0 oder 1

bedeuten, mit der Maßgabe, daß die Verbindung mindestens eine lateral substituierte 1,4-Phenylen-

gruppe enthält. Diese Verbindungen sind Gegenstand der Internationalen Patentanmeldung WO 86/07055 (EP 86903333/0227717).

Die Verbindungen der Formel IV besitzen eine hohe chemische Stabilität. Sie sind farblos und gut mischbar mit allen gebräuchlichen Flüssigkristallen. Ihr Einsatz in flüssigkristallinen Phasen führt zu breiteren Mesophasenbereichen und verbesserten Werten für die Spontanpolarisation in chiral getilteten smektischen Phasen. Die erfindungsgemäßen Phasen eignen sich somit sehr gut für flüssigkristalline Phasen für Displays, die auf dem Prinzip der SSFLC-Technologie beruhen. Ferner eignen sie sich jedoch auch für andere elektrooptische Anzeigevorrichtungen, wie beispielsweise TN-Zellen oder Guest-Host-Zellen. Hier dienen sie neben der Erweiterung des Mesophasenbereichs insbesondere zur Vermeidung von reverse twist und zur Verbesserung der elastischen Konstanten.

$R^1$ und $R^2$ sind vorzugsweise Alkyl- oder Alkoxygruppen mit jeweils 3 bis 10 C-Atomen. Sie sind vorzugsweise geradkettig. Ferner bevorzugt sind jedoch Verbindungen der Formel III, worin eine der Gruppen $R^1$ und $R^2$ eine verzweigte Alkyl- oder Alkoxygruppe bedeutet.

Pyr ist vorzugsweise in 2-Position mit Phe verknüpft.

$Z^1$ ist vorzugsweise —CO—O—, —O—CO—, $—CH_2O—$ oder $—OCH_2—$, insbesondere bevorzugt —O—CO— oder —CO—O—.

m ist vorzugsweise 0.

Die lateral substituierte Phenylengruppe ist vorzugsweise eine durch ein F-Atom (= Phe(F)) oder durch eine CN-Gruppe substituierte 1,4-Phenylengruppe (= Phe(CN)).

Ferner bevorzugt sind Verbindungen mit einer 2,3-Dicyan-1,4-phenylengruppe (= $Phe(CN)_2$), insbesondere Derivate des 2,3-Dicyanhydrochinons (= Phe(2—CN, 3—CN)).

Formel IV umfaßt besonders bevorzugte Verbindungen der Teilformel IVa bis IVm :

| | |
|---|---|
| $R^1$—Pyr—Phe—OCO—Phe(F)—$R^2$ | IVa |
| $R^1$—Pyr—Phe—COO—Phe(F)—$R^2$ | IVb |
| $R^1$—Pyr—Phe—OCO—Phe(CN)—$R^2$ | IVc |
| $R^1$—Pyr—Phe—COO—Phe(CN)—$R^2$ | IVd |
| $R^1$—Phe—Pyr—OCO—Phe(F)—$R^2$ | IVe |
| $R^1$—Phe—Pyr—COO—Phe(F)—$R^2$ | IVf |
| $R^1$—Phe—Pyr—OCO—Phe(CN)—$R^2$ | IVg |
| $R^1$—Phe—Pyr—COO—Phe(CN)—$R^2$ | IVh |
| $R^1$—Pyr—Phe(F)—COO—$A^2$—$R^2$ | IVi |
| $R^1$—Pyr—Phe(F)—OCO—$A^2$—$R^2$ | IVj |
| $R^1$—Pyr—Phe(F)—$OCH_2$—$A^2$—$R^2$ | IVk |
| $R^1$—Phe(F)—Pyr—OCO—$A^2$—$R^2$ | IVl |
| $R^1$—Pyr—Phe—COO—Phe(Z—CN, 3—CN)—$R^2$ | IVm |

In den teilformeln IVa, IVc, IVe und IVm bedeutet $R^2$ vorzugsweise Alkoxy mit 4 bis 10 C-Atomen.

Besonders bevorzugt sind die Verbindungen der Teilformeln IVa, IVb, IVd, IVi, IVj, IVk und IVm, insbesondere bevorzugt diejenigen der Teilformeln IVa und IVb, ferner IVm.

Die Verbindungen der Formel IV können beispielsweise durch Veresterung einer entsprechenden Carbonsäure oder eines ihrer reaktionsfähigen Derivate mit einem entsprechenden Alkohol oder einem seiner reaktionsfähigen Derivate, durch Veretherung einer entsprechenden Hydroxyverbindung, durch Umsetzung eines entsprechenden Amidins oder eines seiner Derivate mit einem 2-Acetyl-alkanaldialkylacetal oder einem seiner reaktionsfähigen Derivate, durch Ersatz der Diazoniumgruppe in einem entsprechenden Diazoniumsalz gegen Cl, F oder CN oder durch Umsetzung einer entsprechenden Chlor- oder Bromverbindung mit einem Cyanid hergestellt werden.

Alle Verbindungen der Formeln II, III und IV sowie diejenigen der entsprechenden Teilformeln sind bevorzugte Komponenten der Formel I für die erfindungsgemäßen smektischen Phasen.

Die bevorzugten Bedeutungen für Gruppen aus Formel I gelten analog auch für die Verbindungen der Formel II, III und IV.

Die erfindungsgemäßen ferroelektrischen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den Verbindungen der Formeln II bis IV,

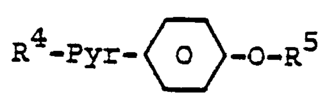

II

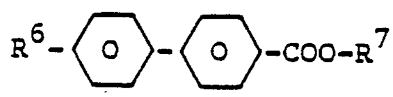

III

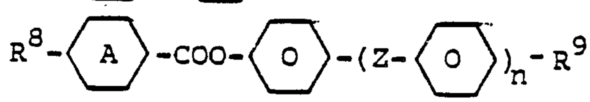

IV

worin $R^4$ und $R^5$ jeweils unabhängig voneinander n-Alkyl mit 5 bis 12 C-Atomen bedeutet und $R^6$, $R^7$, $R^8$ und $R^9$ jeweils unabhängig voneinander geradkettige oder verzweigte, ggf. chirale, Alkyl-, Alkoxy-, Alkoxycarbonyl- oder Alkanoyloxygruppen mit 5 bis 12, insbesondere mit 6 bis 10 C-Atomen bedeutet. Ring a ist 1,4-Phenylen oder trans-1,4-Cyclohexylen. n ist 0 oder 1.

Alle diese Substanzen sind nach literaturbekannten Methoden herstellbar.

Weiterhin bevorzugt sind erfindungsgemäße ferroelektrische Phasen enthaltend mindestens eine Verbindung der Formel V

$$R^1\text{-}Q^1\text{-}A\text{-}(Q^2)_q\text{-}R^2 \qquad \text{V}$$

worin

$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch —O—, —S—, —CO—, $CHCH_3$—O—, —$CHCH_3$—, —CH-Halogen-, CHCN—, —O—CO—, —O—COO—, —CO—O— und/oder —CH=CH— ersetzt sein können,

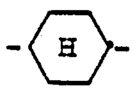, 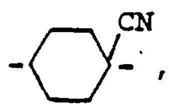, 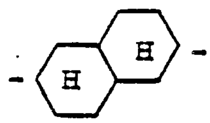,

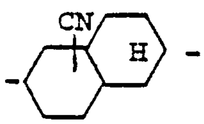, 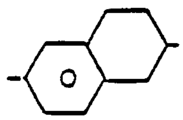, 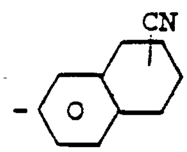,

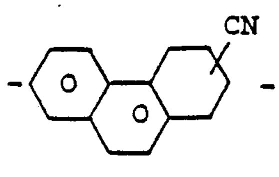, 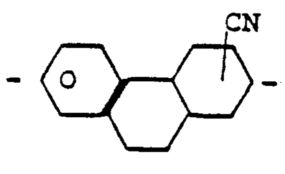,

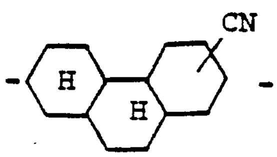, 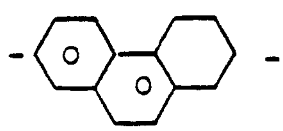,

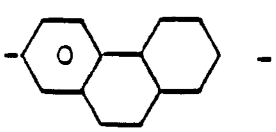, 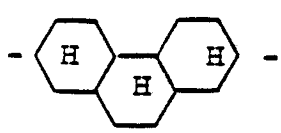,

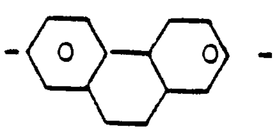, 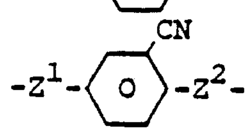 oder

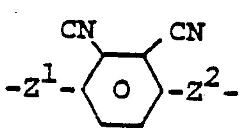

q 0 oder 1,

$Q^1$ und $Q^2$ jeweils unabhängig voneinander, —$(A^0$—$Z^0)_p$—, wobei

$A^0$ unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen durch —O— und/oder —S— und/oder eine —CH—$CH_2$— Gruppierung durch —C=N— ersetzt sein können (Cy), oder unsubstituiertes oder ein- oder mehrfach durch Halogenatome, $CH_3$- und/oder Nitril-Gruppen substituiertes 1,4-Phenylen, worin auch eine oder mehrere CH-Gruppen durch N ersetzt sein können (Ph) bedeutet,

einer der Reste $A^0$ auch 2,6-Naphthylen (Na) oder Tetrahydro-2,6-naphthylen (4H—Na), gegebenenfalls durch Halogen oder CN substituiert,

$Z^0$, $Z^1$ und $Z^2$ jeweils unabhängig voneinander —CO—O—, —O—CO—, —$CH_2O$—, $OCH_2$—, —$CH_2CH_2$—, —$CHCNCH_2$—, —$CH_2$—CHCN— oder eine Einfachbindung, und

p 1, 2 oder 3, oder im Falle A = Tetra- oder Octahydrophenanthren auch O bedeutet, wobei Falle A =

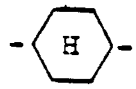, 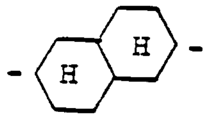, 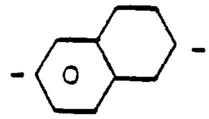

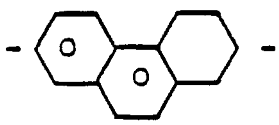, 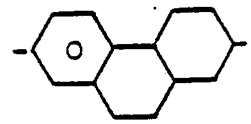

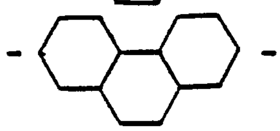 oder 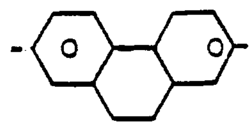

mindestens eine gruppe $Z^0$ —$CHCNCH_2$— oder —$CH_2CHCN$— bedeutet und/oder in mindestens einer der gruppen $R^1$ und $R^2$ mindestens eine $CH_2$-Gruppe durch —CHCN— ersetzt ist.

Die Verbindungen der Formel V können geradkettige oder verzweigte Flügelgruppen $R^1$ und/oder $R^2$ haben. Verbindungen mit verzweigten Flügelgruppen können in Form des Racemates oder als optisch aktive Verbindungen eingesetzt werden. Achirale Basismischungen aus Verbindungen der Formel V und ggf. weiteren achiralen Komponenten können mit chiralen Verbindungen der Formel I oder auch zusätzlich mit anderen chiralen Verbindungen dotiert werden, um chiral getiltete smektische Phasen zu erhalten.

Besonders bevorzugte kleinere Gruppen von Verbindungen sind diejenigen der Formeln V1 bis V18 :

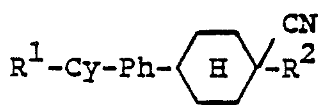 V1

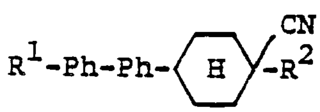 V2

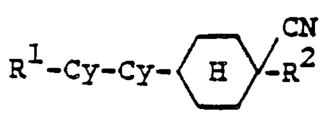 V3

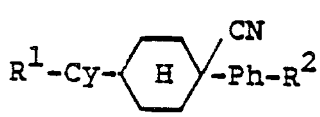 V4

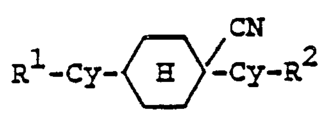 V5

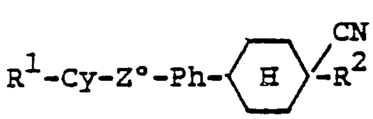 V6

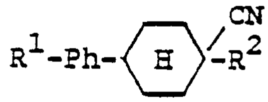

V7

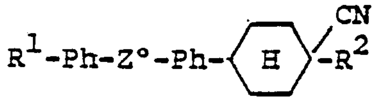

V8

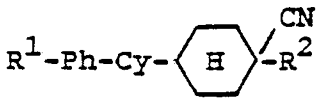

V9

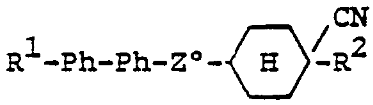

V10

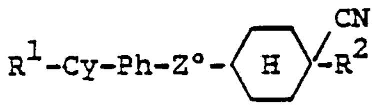

V11

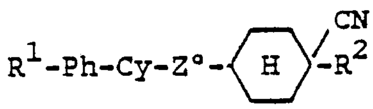

V12

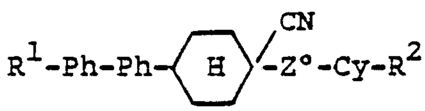

V13

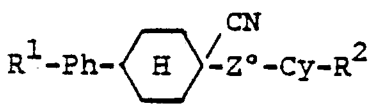

V14

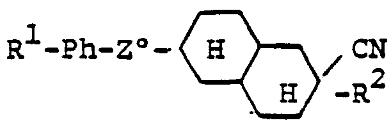

V15

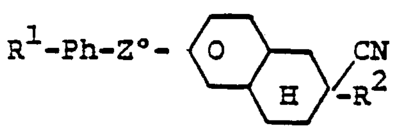

V16

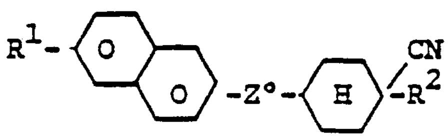

V17

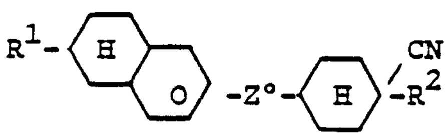

V18

Eine weitere besonders bevorzugte kleinere Gruppe von Verbindungen sind diejenigen der Formeln V19 bis V22 :

$R^1—A^0—Cy—(CH_2)_r—CHCN—C_sH_{2s+1}$ V19

$R^1—A^0—A^0—Cy—(CH_2)_r—CHCN—C_sH_{2s+1}$ V20

$R^1—A^0—A^0—CHCN—CH_2—Cy—R^2$ V21

$R^1—A^0—A^0—CH_2—CHCN—Cy—R^2$ V22

worin r 0, 1, 2 oder 3 bedeutet und (r + s) 1 bis 14 ist.

Verbindungen der Formel V, die keine $S_c$-Phasen aufweisen, eigenen sich ebenfalls als Komponenten erfindungsfemäßer smektischer Phasen.

Alle Verbindungen der Formel V werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Formel V umfaßt zum überwiegenden Teil bekannte Verbindungen, wie beispielsweise die bevorzugten, in DOS 32 31 707, 33 19 781, 33 20 024, 34 07 013, 34 43 029, 33 32 690, 33 32 691, 33 32 692, 29 33 563, 28 53 728, 26 13 293, 34 01 320, 31 36 624, 30 40 632, 32 05 766, 22 40 864, 29 37 700, 34 10 734, 33 24 686, EP-OS 0 085 995, EP-OS 0 084 194, DD 116 732, FR 24 25 469, FR 24 19 966, USP 4 237 026, USP 3 953 491, USP 4, 225, 454 bzw. in H. J. Deutscher et al., J. prakt. Chemie, 321, 569 (1979) und J. C. Dubois et al. Mol. Cryst. Liq. Cryst. 47, 193 (1978) beschriebenen Verbindungen.

Als Komponenten der erfindungsgemäßen Phasen kommen ferner Verbindungen der Formel

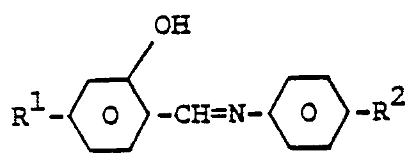

in Frage, worin $R^1$ und $R^2$ die bei Formel V angegebene Bedeutung haben.

Die erfindungsgemäßen Phasen enthalten vorzugsweise mindestens drei, insbesondere mindestens fünf Verbindungen der Formel I. Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Ferner bevorzugt sind erfindungsgemäße Phasen, die im wesentlichen ausschließlich optisch aktive Komponenten enthalten. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vj :

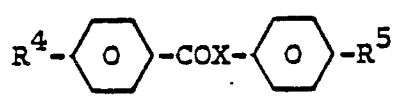 Va

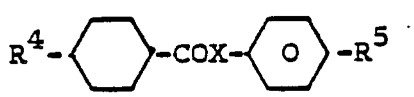 Vb

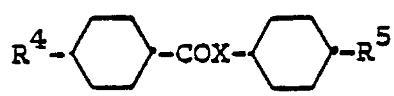 Vc

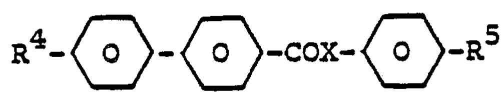 Vd

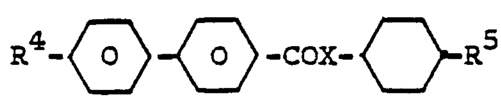 Ve

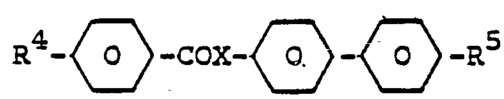 Vf

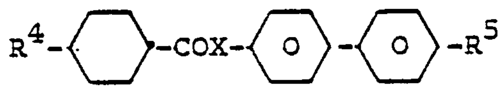

Vg

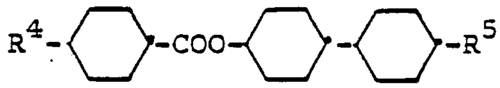

Vh

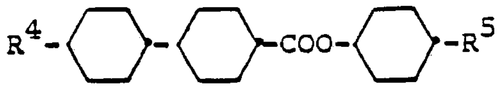

Vi

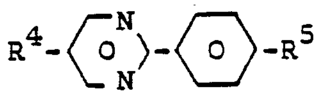

Vj

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X ist vorzugsweise 0.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunktserniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

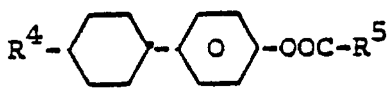

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement A, B oder C.

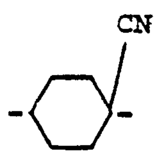

A

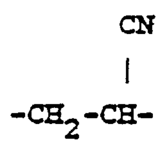

B

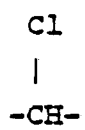

C

Bevorzugte Verbindungen dieser Art entsprechen den Formeln VIa, VIb und VIc :

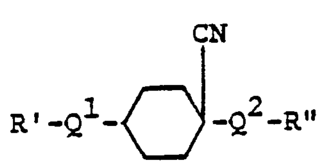

VIa

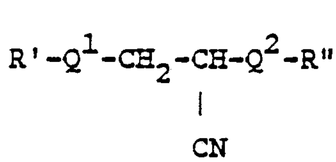

VIb

$$R'-Q^3-Q^4-R'''$$ VIc

R′ und R″ bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4′-Biphenylyl, 4-(trans-4-Cyclohexyl)-phenyl, trans, trans-4,4′-Bicyclohexyl oder eine der Gruppen $Q^1$ und $Q^2$ auch eine Einfachbindung.

$Q^3$ und $Q_4$ bedeuten jeweils 1,4-Phenylen, 4,4′-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R‴ ist ein optisch aktiver Rest mit einem asymmetrischen Kohlenstoffatom der Struktur $-\overset{\overset{Cl}{|}}{C}H^*-$ oder $-\overset{\overset{CN}{|}}{C}H^*-$. Besonders bevorzugte Verbindungen der Formel VIc sind diejenigen der Formel VIc′ :

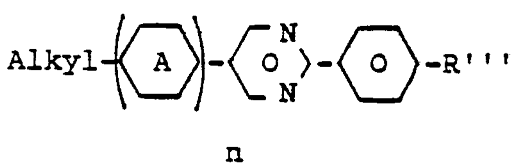

VIc'

worin A 1,4-Phenylen oder trans-1,4-Cyclohexylen und n 0 oder 1 bedeutet.

Die Herstellung der erfindungsgemäßem Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent ; alle Temperaturangaben sind in Grad Celsius angegeben. Die Werte für die spontane Polarisation gelten für Raumtemperatur. Es bedeuten ferner : K : Kristallin-fester Zustand, S : Smektische Phase (der Index kennzeichnet den Phasentyp), N : Nematischer Zustand, Ch : Cholesterische Phase, I : Isotrope Phase. Die zwischen zwei Symbolen stehende Zahl gibt die Umwandlungstemperaturen in Grad Celsius an. « Übliche Aufarbeitung » bedeutet : man gibt Wasser hinzu, extrahiert mit Methylenchlorid, trennt ab, trocknet die organische Phase, dampft ein und reinigt das Produkt durch Kristallisation und/oder Chromatographie.

## Beispiel 1

Eine flüssigkristalline Phase bestehend aus

18 % 4-(5-Heptylpyrimidin-2-yl)-phenyl-(p-pentylbenzyl)-ether,
26 % 4-(5-Heptylpyrimidin-2-yl)-phenyl-(p-hexylbenzyl)-ether,
13 % 4-(5-Nonylpyrimidin-2-yl)-phenyl-(p-pentylbenzyl)-ether,
4 % 4-(5-Nonylpyrimidin-2-yl)-phenyl-(p-hexylbenzyl)-ether,
24 % 2-p-Nonyloxyphenyl-5-nonylpyrimidin,
8 % p-(5-Pentylpyrimidin-2-yl)-benzoesäure-[p-(5-nonylpyrimidin-2-yl)-phenyl]-ester und
7 % p-(5-Heptylpyrimidin-2-yl)-benzoesäure-[p-(5-heptylpyrimidin-2-yl)-phenyl]-ester

hat $K/S_c$ 13°, $S_c/N$ 64° und N/I 124°.

## Beispiel 2

Eine flüssigkristalline Phase bestehend aus

18 % 4-(5-Heptylpyrimidin-2-yl)-phenyl-(p-pentylbenzyl)-ether,
22 % 4-(5-Heptylpyrimidin-2-yl)-phenyl-(p-hexylbenzyl)-ether,
13 % 4-(5-Nonylpyrimidin-2-yl)-phenyl-(p-pentylbenzyl)-ether,
10 % 4-(5-Nonylpyrimidin-2-yl)-phenyl-(p-hexylbenzyl)-ether,
21 % 2-p-Undecyloxyphenyl-5-heptylpyrimidin,
5 % 2-(p-Pentylphenyl)-pyrimidin-5-yl-(p-octylbenzyl)-ether,
5 % 2-(p-Hexylphenyl)-pyrimidin-5-yl-(p-nonylbenzyl)-ether und
6 % 4-(5-Octylpyrimidin-2-yl)-phenyl-trans-4-pentylcyclohexylmethyl-ether

hat $K/S_c$ 6°, $S_c/N$ 63,5° und N/I 96°.

Beispiel 3

Eine flüssigkristalline Phase bestehend aus

12 % 4-(5-Heptylpyrimidin-2-yl)-phenyl-(p-pentylbenzyl)-ether,
15 % 4-(5-Heptylpyrimidin-2-yl)-phenyl-(p-hexylbenzyl)-ether,
10 % 4-(5-Nonylpyrimidin-2-yl)-phenyl-(p-hexylbenzyl)-ether,
8 % 2-p-Nonyloxyphenyl-5-nonylpyrimidin,
16 % 2-p-Decyloxyphenyl-5-octylpyrimidin,
13 % p-(5-Pentylpyrimidin-2-yl)-benzoesäure-[p-(5-hexylpyrimidin-2-yl)-phenyl]-ester,
12 % 2-(p-Pentylphenyl)-pyrimidin-2-yl-(p-nonylbenzyl)-ether,
5 % 2-p-Undecyloxyphenyl-5-heptylpyrimidin,
4 % 4-(5-Octylpyrimidin-2-yl)-phenyl-trans-4-hexylcyclohexylmethyl-ether und
5 % 4-(5-Nonylpyrimidin-2-yl)-phenyl-trans-4-pentylcyclohexylmethyl-ether

hat $K/S_c$ — 1°, $S_c/N$ 63° und N/I 94°.

Beispiel 4

Eine flüssigkristalline Phase bestehend aus

20 % 4-(5-Hexylpyrimidin-2-yl)-phenyl-(p-hexylbenzyl)-ether,
15 % 4-(5-Heptylpyrimidin-2-yl)-phenyl-(p-pentylbenzyl)-ether,
10 % 4-(5-Nonylpyrimidin-2-yl)-phenyl-(p-hexylbenzyl)-ether,
4 % 4-(5-Heptylpyrimidin-2-yl)-phenyl-(p-hexylbenzyl)-ether,
16 % 4-(5-Nonylpyrimidin-2-yl)-phenyl-(p-pentylbenzyl)-ether,
10 % 2-p-Nonyloxyphenyl-5-heptylpyrimidin,
7 % 2-p-Nonyloxyphenyl-5-nonylpyrimidin,
8 % trans-4-Pentylcyclohexancarbonsäure-(p-hexyloxyphenyl)-ester und
10 % p-Decyloxybenzoesäure-(p-heptylphenyl)-ester

hat $K/S_c$ 0°, $S_c/N$ 79° und N/I 87°.

Beispiel 5

Eine flüssigkristalline Phase bestehend aus

18 % 4-(5-Hexylpyrimidin-2-yl)-phenyl-(p-hexylbenzyl)-ether,
14 % 4-(5-Heptylpyrimidin-2-yl)-phenyl-(p-pentylbenzyl)-ether,
10 % 4-(5-Hexylpyrimidin-2-yl)-phenyl-(p-pentylbenzyl)-ether,
10 % 4-(5-Nonylpyrimidin-2-yl)-phenyl-(p-hexylbenzyl)-ether,
20 % 4-(5-Heptylpyrimidin-2-yl)-phenyl-(p-hexylbenzyl)-ether,
10 % 4-(5-Nonylpyrimidin-2-yl)-phenyl-(p-pentylbenzyl)-ether,
6 % 2-p-Decyloxyphenyl-5-octylpyrimidin,
7 % p-Decyloxybenzoesäure-(p-heptylphenyl)-ester und
5 % p-(p-Decyloxybenzoyloxy)-benzoesäure-(p-butyl-oxyphenyl)-ester

hat $K/S_c$ 12°, $S_c/N$ 96° und N/I 107°.

Beispiel 6

Eine flüssigkristalline Phase bestehend aus

27 % 4-(5-Hexylpyrimidin-2-yl)-phenyl-(p-hexylbenzyl)-ether,
14 % 4-(5-Heptylpyrimidin-2-yl)-phenyl-(p-pentylbenzyl)-ether,
7 % 4-(5-Hexylpyrimidin-2-yl)-phenyl-(p-pentylbenzyl)-ether,
2 % 4-(5-Nonylpyrimidin-2-yl)-phenyl-(p-hexylbenzyl)-ether,
18 % 4-(5-Heptylpyrimidin-2-yl)-phenyl-(p-hexylbenzyl)-ether,
7 % 4-(5-Nonylpyrimidin-2-yl)-phenyl-(p-pentylbenzyl)-ether,
4 % 2-p-Heptyloxyphenyl-5-octylpyrimidin,
2 % trans-4-Pentylcyclohexancarbonsäure-(p-hexyloxy-phenyl)-ester,
10 % p-Pentylbenzoesäure-(4′-pentylbiphenyl-4-yl)-ester und
9 % p-Hexylbenzoesäure-(4′-pentylbiphenyl-4-yl)-ester

hat $K/S_c$ 9°, $S_c/N$ 82° und N/I 116°.

Beispiel 7

Eine flüssigkristalline Phase bestehend aus

16 % 4-(5-Hexylpyrimidin-2-yl)-phenyl-(p-pentylbenzyl)-ether,
26 % 4-(5-Heptylpyrimidin-2-yl)-phenyl-(p-pentylbenzyl)-ether,
36 % 4-(5-Heptylpyrimidin-2-yl)-phenyl-(p-hexylbenzyl)-ether,
18 % 4-(5-Nonylpyrimidin-2-yl)-phenyl-(p-pentylbenzyl)-ether und
4 % 4-(5-Nonylpyrimidin-2-yl)-phenyl-(p-hexylbenzyl)-ether

hat K/$S_c$ 8°, $S_c$/N 84,5° und N/I 104°.

Beispiel 8

Eine flüssigkristalline Phase bestehend aus

30 % 4-(5-Hexylpyrimidin-2-yl)-phenyl-(p-hexylbenzyl)-ether,
15 % 4-(5-Heptylpyrimidin-2-yl)-phenyl-(p-pentylbenzyl)-ether,
8 % 4-(5-Hexylpyrimidin-2-yl)-phenyl-(p-pentylbenzyl)-ether,
2 % 4-(5-Nonylpyrimidin-2-yl)-phenyl-(p-hexylbenzyl)-ether,
21 % 4-(5-Heptylpyrimidin-2-yl)-phenyl-(p-hexylbenzyl)-ether,
8 % 4-(5-Nonylpyrimidin-2-yl)-phenyl-(p-pentylbenzyl)-ether und
16 % 2-p-Nonyloxyphenyl-5-nonylpyrimidin

hat K/$S_c$ — 5°, $S_c$/N 81° und N/I 99°.

Beispiel 9

Eine flüssigkristalline Phase bestehend aus

27 % 4-(5-Hexylpyrimidin-2-yl)-phenyl-(p-hexylbenzyl)-ether,
14 % 4-(5-Heptylpyrimidin-2-yl)-phenyl-(p-pentylbenzyl)-ether,
7 % 4-(5-Hexylpyrimidin-2-yl)-phenyl-(p-pentylbenzyl)-ether,
2 % 4-(5-Nonylpyrimidin-2-yl)-phenyl-(p-hexylbenzyl)-ether,
18 % 4-(5-Heptylpyrimidin-2-yl)-phenyl-(p-hexylbenzyl)-ether,
7 % 4-(5-Nonylpyrimidin-2-yl)-phenyl-(p-pentylbenzyl)-ether,
14 % 2-p-Nonyloxyphenyl-5-nonylpyrimidin und
10 % R-4-(5-Hexylpyrimidin-2-yl)-phenyl-2-chlorpropionat

hat K/$S^*_c$ — 4°, $S^*_c$/Ch 64,7° und Ch/I 92,5°.

Die Werte der spontanen Polarisation (nC/cm²) und die Pitchhöhe (μm) dieser Phase sind in der folgenden Tabelle für verschiedene Temperaturen angeben :

| Temperatur [°C] | Spontane Polarisation | Pitch |
|---|---|---|
| 60 | 2,4 | 14 |
| 50 | 4,9 | 13,5 |
| 40 | 6,1 | 12 |
| 20 | 7,3 | 11 |
| 8 | 9,0 | 10 |

Beispiel 10

Eine flüssigkristalline Phase bestehend aus

31 % 2-p-Nonyloxyphenyl-5-nonylpyrimidin
9 % 2-p-Decyloxyphenyl-5-octylpyrimidin
14 % 2-p-Octyloxyphenyl-5-octylpyrimidin
18 % 2-p-Nonyloxyphenyl-5-heptylpyrimidin
13 % 2-p-Heptyloxyphenyl-5-heptylpyrimidin
10 % 2-Fluor-4-decyloxybenzoesäure-(4-(5-octylpyrimidin-2-yl)-phenyl)-ester
5 % 2-Fluor-4-hexyloxybenzoesäure-(4-(5-decylpyrimidin-2-yl)-phenyl)-ester

hat K/$S_c$ 7°, $S_c$/$S_A$ 57°, $S_A$/N 67°, N/I 70,5°.

Beispiel 11

Eine flüssigkristalline Phase bestehend aus

28 % 2-p-Nonyloxyphenyl-5-nonylpyrimidin
3 % 2-p-Nonyloxyphenyl-5-octylpyrimidin
25 % 2-p-Heptyloxyphenyl-5-octylpyrimidin
10 % 2-p-Heptyloxy-5-heptylpyrimidin
5 % 2-p-Decyloxy-5-heptylpyrimidin
9 % 2-Fluor-4-decyloxybenzoesäure-(4-(5-octylpyrimidin-2-yl)-phenyl)-ester
10 % 3-Fluor-4-heptyloxybenzoesäure-(4-(5-heptylpyrimidin-2-yl)-phenyl)-ester
10 % 4-(5-Decylpyrimidin-2-yl)-benzoesäure-(2-fluor-4-nonylphenyl)-ester

hat K/$S_c$ 13°, $S_c$/$S_A$ 64°, $S_A$/N 68°, N/I 83°.

Beispiel 12

Eine flüssigkristalline Phase bestehend aus

30 % 2-p-Nonyloxyphenyl-5-nonylpyrimidin
15 % 2-p-Decyloxyphenyl-5-octylpyrimidin
15 % 2-p-Octyloxyphenyl-5-octylpyrimidin
12 % 2-p-Nonyloxyphenyl-5-heptylpyrimidin
10 % 4-(5-Octylpyrimidin-2-yl)-benzoesäure-(3-fluor-4-hexylphenyl)-ester
3 % 4-(5-Decylpyrimidin-2-yl)-benzoesäure-(2-cyan-4-decylphenyl)-ester
6 % 2-Fluor-4-heptylbenzoesäure-(2-(4-heptylphenyl)-pyrimidin-5-yl)-ester
9 % 3-Fluor-4-hexylbenzoesäure-(2-(4-octylphenyl)-pyrimidin-5-yl)-ester

hat K/$S_c$ 16°, $S_c$/$S_A$ 59°, $S_A$/N 63°, N/I 79°.

Beispiel 13

Eine flüssigkristalline Phase bestehend aus

30 % 2-p-Nonyloxyphenyl-5-nonylpyrimidin
15 % 2-p-Decyloxyphenyl-5-octylpyrimidin
20 % 2-p-Octyloxyphenyl-5-octylpyrimidin
15 % 2-p-Nonyloxyphenyl-5-heptylpyrimidin
10 % 2-p-Heptyloxyphenyl-5-heptylpyrimidin
10 % 4-Octylbenzoesäure-(2-fluor-4-(5-octyl-pyrimidin-2-yl)-phenyl)-ester

hat K/$S_c$ 4°, $S_c$/$S_A$ 22°, $S_A$/N 65°, N/I 74°.

Beispiel 14

Eine flüssigkristalline Phase bestehend aus

33 % 4-(5-Hexylpyrimidin-2-yl)-phenyl-(p-hexylbenzyl)-ether
15 % 4-(5-Heptylpyrimidin-2-yl)-phenyl-(p-pentylbenzyl)-ether
10 % 4-(5-Hexylpyrimidin-2-yl)-phenyl-(p-pentylbenzyl)-ether
3 % 4-(5-Nonylpyrimidin-2-yl)-phenyl-(p-hexylbenzyl)-ether

24 % 4-(5-Heptylpyrimidin-2-yl)-phenyl-(p-hexylbenzyl)-ether
10 % 2-Fluor-4-heptyloxybenzoesäure-(4-5-octyl-pyrimidin-2-yl)-phenyl)-ester
5 % 4-(5-Decylpyrimidin-2-yl)-benzoesäure-(3-fluor-4-octylphenyl)-ester

hat K/$S_c$ 3°, $S_c$/N 75°, N/I 98°.

Beispiel 15

Eine flüssigkristalline Phase bestehend aus

32 % 4-(5-Hexylpyrimidin-2-yl)-phenyl-(p-hexylbenzyl)-ether
16 % 4-(5-Heptylpyrimidin-2-yl)-phenyl-(p-pentylbenzyl)-ether
9 % 4-(5-Hexylpyrimidin-2-yl)-phenyl-(p-pentylbenzyl)-ether
2 % 4-(5-Nonylpyrimidin-2-yl)-phenyl-(p-hexylbenzyl)-ether
18 % 4-(5-Nonylpyrimidin-2-yl)-phenyl-(p-pentylbenzyl)-ether
13 % 3-Fluor-4-heptyloxybenzoesäure-(4-(5-heptylpyrimidin-2-yl)-phenyl)-ester
5 % 4-(5-Decylpyrimidin-2-yl)-benzoesäure-(2-fluor-4-nonylphenyl)-ester
5 % 4-(5-Nonylpyrimidin-2-yl)-benzoesäure-(2-cyano-4-octylphenyl)-ester

hat K/$S_c$ — 1°, $S_c$/N 77°, N/I 94°.

Beispiel 16

Eine flüssigkristalline Phase bestehend aus

17 % 4-(5-Heptylpyrimidin-2-yl)-phenyl-(p-pentylbenzyl)-ether
25 % 4-(5-Heptylpyrimidin-2-yl)-phenyl-(p-hexylbenzyl)-ether
13 % 4-(Nonylpyrimidin-2-yl)-phenyl-(p-pentylbenzyl)-ether
4 % 4-(Nonylpyrimidin-2-yl)-phenyl-(p-hexylbenzyl)-ether
11 % 2-Fluor-4-heptylbenzoesäure-(2-(4-nonylphenyl)-pyrimidin-5-yl)-ester
10 % 4-Octylbenzoesäure-(2-fluor-4-(5-decylpyrimidin-2-yl)-phenyl)-ester
15 % 2-Fluor-4-heptyloxybenzoesäure-(4-(5-heptylpyrimidin-2-yl)-phenyl)-ester
5 % 4-(5-Nonylpyrimidin-2-yl)-benzoesäure-(2-fluor-4-nonylphenyl)-ester

hat K/$S_c$ 5°, $S_c$/N 75°, N/I 97°.

Beispiel 17

Eine flüssigkristalline Phase bestehend aus

33 % 4-(5-Hexylpyrimidin-2-yl)-phenyl-(p-hexylbenzyl)-ether
18 % 4-(5-Heptylpyrimidin-2-yl)-phenyl-(p-pentylbenzyl)-ether
10 % 4-(5-Hexylpyrimidin-2-yl)-phenyl-(p-pentylbenzyl)-ether
3 % 4-(5-Nonylpyrimidin-2-yl)-phenyl-(p-hexylbenzyl)-ether
25 % 4-(5-Heptylpyrimidin-2-yl)-phenyl-(p-hexylbenzyl)-ether
4 % 3-Fluor-4-octylbenzoesäure-(5-(4-octylphenyl)-pyrimidin-2-yl)-ester
7 % 4-Heptylbenzoesäure-(2-fluor-4-(5-decylpyrimidin-2-yl)-phenyl)-ester

hat K/$S_c$ 10°, $S_c$/N 77°, N/I 100°.

Beispiel 18

Ein Gemisch von 0,65 g 5-Methoxy-2-(4-n-Octylphenyl)-pyrimidin [erhältlich durch Kondensation von Methyl-β-hydroxy-α-methoxy-acrylat mit 4-n-Octylbenzamidinhydrochlorid, Umsetzung des erhaltenen 4-Hydroxy-5-methoxy-pyrimidins mit $P_2S_5$ und Reduktion des erhaltenen 4-Mercaptopyrimidins mit Raney-Nickel nach J. H. Chesterfield et al., J. Chem. Soc. 4590 (1960)] und 1,0 g KOH in 15 ml Ethylenglykol wird 10 Stunden gekocht. Die abgekühlte Lösung wird mit 10 ml Wasser versetzt, mit Eisessig angesäuert und fünfmal mit Ether extrahiert. Nach üblicher Aufarbeitung erhält man 5-Hydroxy-2-(4-n-octylphenyl)-pyrimidin. (Die anderen Homologen sind analog erhältlich). Anschließend werden 0,26 g des erhaltenen 5-Hydroxy-2-(4-n-octylphenyl)-pyrimidin, 0,25 g 4-n-Heptylbenzoylchlorid und 0,15 g Kaliumcarbonat in 2,5 ml Dimethylformamid 12 Stunden unter Rühren auf 100° erhitzt. Man saugt vom Salz ab, engt zum Rückstand ein, nimmt in Ether auf und wäscht mit Wasser neutral. Die übliche Aufarbeitung ergibt 4-n-Heptylbenzoesäure-[2-(4-n-Octylphenyl)-pyrimidin-5-yl]-ester.

Analog werden hergestellt :

4-Propylbenzoesäure-(2-(4-propylphenyl)-pyrimidin-5-yl)-ester
4-Propylbenzoesäure-(2-(4-pentylphenyl)-pyrimidin-5-yl)-ester
4-Propylbenzoesäure-(2-(4-hexylphenyl)-pyrimidin-5-yl)-ester
4-Propylbenzoesäure-(2-(4-heptylphenyl)-pyrimidin-5-yl)-ester
4-Propylbenzoesäure-(2-(4-octylphenyl)-pyrimidin-5-yl)-ester
4-Propylbenzoesäure-(2-(4-nonylphenyl)-pyrimidin-5-yl)-ester
4-Propylbenzoesäure-(2-(4-decylphenyl)-pyrimidin-5-yl)-ester

4-Hexylbenzoesäure-(2-(4-propylphenyl)-pyrimidin-5-yl)-ester
4-Hexylbenzoesäure-(2-(4-pentylphenyl)-pyrimidin-5-yl)-ester
4-Hexylbenzoesäure-(2-(4-hexylphenyl)-pyrimidin-5-yl)-ester
4-Hexylbenzoesäure-(2-(4-heptylphenyl)-pyrimidin-5-yl)-ester
4-Hexylbenzoesäure-(2-(4-octylphenyl)-pyrimidin-5-yl)-ester
4-Hexylbenzoesäure-(2-(4-nonylphenyl)-pyrimidin-5-yl)-ester
4-Hexylbenzoesäure-(2-(4-decylphenyl)-pyrimidin-5-yl)-ester

4-Heptylbenzoesäure-(2-(4-propylphenyl)-pyrimidin-5-yl)-ester
4-Heptylbenzoesäure-(2-(4-pentylphenyl)-pyrimidin-5-yl)-ester
4-Heptylbenzoesäure-(2-(4-hexylphenyl)-pyrimidin-5-yl)-ester
4-Heptylbenzoesäure-(2-(4-heptylphenyl)-pyrimidin-5-yl)-ester
4-Heptylbenzoesäure-(2-(4-octylphenyl)-pyrimidin-5-yl)-ester
4-Heptylbenzoesäure-(2-(4-nonylphenyl)-pyrimidin-5-yl)-ester
4-Heptylbenzoesäure-(2-(4-decylphenyl)-pyrimidin-5-yl)-ester

4-Octylbenzoesäure-(2-(4-propylphenyl)-pyrimidin-5-yl)-ester
4-Octylbenzoesäure-(2-(4-pentylphenyl)-pyrimidin-5-yl)-ester
4-Octylbenzoesäure-(2-(4-hexylphenyl)-pyrimidin-5-yl)-ester
4-Octylbenzoesäure-(2-(4-heptylphenyl)-pyrimidin-5-yl)-ester
4-Octylbenzoesäure-(2-(4-octylphenyl)-pyrimidin-5-yl)-ester
4-Octylbenzoesäure-(2-(4-nonylphenyl)-pyrimidin-5-yl)-ester
4-Octylbenzoesäure-(2-(4-decylphenyl)-pyrimidin-5-yl)-ester

4-Nonylbenzoesäure-(2-(4-propylphenyl)-pyrimidin-5-yl)-ester
4-Nonylbenzoesäure-(2-(4-pentylphenyl)-pyrimidin-5-yl)-ester
4-Nonylbenzoesäure-(2-(4-hexylphenyl)-pyrimidin-5-yl)-ester
4-Nonylbenzoesäure-(2-(4-heptylphenyl)-pyrimidin-5-yl)-ester
4-Nonylbenzoesäure-(2-(4-octylphenyl)-pyrimidin-5-yl)-ester
4-Nonylbenzoesäure-(2-(4-nonylphenyl)-pyrimidin-5-yl)-ester
4-Nonylbenzoesäure-(2-(4-decylphenyl)-pyrimidin-5-yl)-ester

4-Decylbenzoesäure-(2-(4-propylphenyl)-pyrimidin-5-yl)-ester
4-Decylbenzoesäure-(2-(4-pentylphenyl)-pyrimidin-5-yl)-ester
4-Decylbenzoesäure-(2-(4-hexylphenyl)-pyrimidin-5-yl)-ester
4-Decylbenzoesäure-(2-(4-heptylphenyl)-pyrimidin-5-yl)-ester
4-Decylbenzoesäure-(2-(4-octylphenyl)-pyrimidin-5-yl)-ester
4-Decylbenzoesäure-(2-(4-nonylphenyl)-pyrimidin-5-yl)-ester
4-Decylbenzoesäure-(2-(4-decylphenyl)-pyrimidin-5-yl)-ester

Beispiel 19

Ein Gemisch von 10,2 g 5-Hydroxy-2-(4-n-hexylphenyl)-pyrimidin, 8,6 g 4-n-Propylbenzylbromid, 8,6 g Kaliumcarbonat und 50 ml Dimethylformamid wird 10 Stunden auf 90° erwärmt. Übliche Aufarbeitung liefert (2-(4-n-Hexylphenyl)-pyrimidin-5-yl)-(p-n-propylbenzyl)-ether.

Analog werden hergestellt :

(2-(4-Butylphenyl)-pyrimidin-5-yl)-(p-propylbenzyl)-ether
(2-(4-Butylphenyl)-pyrimidin-5-yl)-(p-pentylbenzyl)-ether
(2-(4-Butylphenyl)-pyrimidin-5-yl)-(p-hexylbenzyl)-ether
(2-(4-Butylphenyl)-pyrimidin-5-yl)-(p-heptylbenzyl)-ether
(2-(4-Butylphenyl)-pyrimidin-5-yl)-(p-octylbenzyl)-ether
(2-(4-Butylphenyl)-pyrimidin-5-yl)-(p-nonylbenzyl)-ether
(2-(4-Butylphenyl)-pyrimidin-5-yl)-(p-decylbenzyl)-ether

(2-(4-Pentylphenyl)-pyrimidin-5-yl)-(p-propylbenzyl)-ether
(2-(4-Pentylphenyl)-pyrimidin-5-yl)-(p-pentylbenzyl)-ether
(2-(4-Pentylphenyl)-pyrimidin-5-yl)-(p-hexylbenzyl)-ether
(2-(4-Pentylphenyl)-pyrimidin-5-yl)-(p-heptylbenzyl)-ether
(2-(4-Pentylphenyl)-pyrimidin-5-yl)-(p-octylbenzyl)-ether
(2-(4-Pentylphenyl)-pyrimidin-5-yl)-(p-nonylbenzyl)-ether
(2-(4-Pentylphenyl)-pyrimidin-5-yl)-(p-decylbenzyl)-ether

(2-(4-Hexylphenyl)-pyrimidin-5-yl)-(p-propylbenzyl)-ether
(2-(4-Hexylphenyl)-pyrimidin-5-yl)-(p-pentylbenzyl)-ether
(2-(4-Hexylphenyl)-pyrimidin-5-yl)-(p-hexylbenzyl)-ether
(2-(4-Hexylphenyl)-pyrimidin-5-yl)-(p-heptylbenzyl)-ether
(2-(4-Hexylphenyl)-pyrimidin-5-yl)-(p-octylbenzyl)-ether
(2-(4-Hexylphenyl)-pyrimidin-5-yl)-(p-nonylbenzyl)-ether
(2-(4-Hexylphenyl)-pyrimidin-5-yl)-(p-decylbenzyl)-ether

(2-(4-Heptylphenyl)-pyrimidin-5-yl)-(p-propylbenzyl)-ether
(2-(4-Heptylphenyl)-pyrimidin-5-yl)-(p-pentylbenzyl)-ether
(2-(4-Heptylphenyl)-pyrimidin-5-yl)-(p-hexylbenzyl)-ether
(2-(4-Heptylphenyl)-pyrimidin-5-yl)-(p-heptylbenzyl)-ether
(2-(4-Heptylphenyl)-pyrimidin-5-yl)-(p-octylbenzyl)-ether
(2-(4-Heptylphenyl)-pyrimidin-5-yl)-(p-nonylbenzyl)-ether
(2-(4-Heptylphenyl)-pyrimidin-5-yl)-(p-decylbenzyl)-ether

(2-(4-Octylphenyl)-pyrimidin-5-yl)-(p-propylbenzyl)-ether
(2-(4-Octylphenyl)-pyrimidin-5-yl)-(p-pentylbenzyl)-ether
(2-(4-Octylphenyl)-pyrimidin-5-yl)-(p-hexylbenzyl)-ether
(2-(4-Octylphenyl)-pyrimidin-5-yl)-(p-heptylbenzyl)-ether
(2-(4-Octylphenyl)-pyrimidin-5-yl)-(p-octylbenzyl)-ether
(2-(4-Octylphenyl)-pyrimidin-5-yl)-(p-nonylbenzyl)-ether
(2-(4-Octylphenyl)-pyrimidin-5-yl)-(p-decylbenzyl)-ether

(2-(4-Nonylphenyl)-pyrimidin-5-yl)-(p-propylbenzyl)-ether
(2-(4-Nonylphenyl)-pyrimidin-5-yl)-(p-pentylbenzyl)-ether
(2-(4-Nonylphenyl)-pyrimidin-5-yl)-(p-hexylbenzyl)-ether
(2-(4-Nonylphenyl)-pyrimidin-5-yl)-(p-heptylbenzyl)-ether
(2-(4-Nonylphenyl)-pyrimidin-5-yl)-(p-octylbenzyl)-ether
(2-(4-Nonylphenyl)-pyrimidin-5-yl)-(p-nonylbenzyl)-ether
(2-(4-Nonylphenyl)-pyrimidin-5-yl)-(p-decylbenzyl)-ether

(2-(4-Decylphenyl)-pyrimidin-5-yl)-(p-propylbenzyl)-ether
(2-(4-Decylphenyl)-pyrimidin-5-yl)-(p-pentylbenzyl)-ether
(2-(4-Decylphenyl)-pyrimidin-5-yl)-(p-hexylbenzyl)-ether
(2-(4-Decylphenyl)-pyrimidin-5-yl)-(p-heptylbenzyl)-ether
(2-(4-Decylphenyl)-pyrimidin-5-yl)-(p-octylbenzyl)-ether
(2-(4-Decylphenyl)-pyrimidin-5-yl)-(p-nonylbenzyl)-ether
(2-(4-Decylphenyl)-pyrimidin-5-yl)-(p-decylbenzyl)-ether

## Beispiel 20

14,2 g 4-(5-Hexylpyrimidin-2-yl)-benzoesäure (erhältlich aus 4-(5-Hexylpyrimidin-2-yl)-benzonitril durch Verseifung) werden mit 10 g Thionylchlorid in 120 ml Toluol bis zum Ende der Chlorwasserstoffentwicklung zum Rückfluß erhitzt. Unter verminderten Druck befreit man von flüchtigen Anteilen. Der Rückstand wird in 180 ml Dichlormethan aufgenommen und mit 10 ml Triethylamin und 14,2 g 4-(5-Octylpyrimidin-2-yl)-phenol (erhältlich aus 4-Hydroxybenzamidinhydrochlorid und α-Octyl-β-hydroxyacrylsäuremethylester) versetzt. Nach 20 Std. Rühren bei 20° filtriert man ausgeschiedenes Triethylammoniumchlorid ab und engt das Filtrat ein. Es kristallisiert 4-(5-Hexylpyrimidin-2-yl)-benzoesäure-(4-(5-octylpyrimidin-2-yl)-phenyl)-ester.

Analog hierzu werden hergestellt :

4-(5-Propylpyrimidin-2-yl)-benzoesäure-(4-(5-propylpyrimidin-2-yl)-phenyl)-ester
4-(5-Propylpyrimidin-2-yl)-benzoesäure-(4-(5-pentylpyrimidin-2-yl)-phenyl)-ester
4-(5-Propylpyrimidin-2-yl)-benzoesäure-(4-(5-hexylpyrimidin-2-yl)-phenyl)-ester
4-(5-Propylpyrimidin-2-yl)-benzoesäure-(4-(5-heptylpyrimidin-2-yl)-phenyl)-ester

4-(5-Propylpyrimidin-2-yl)-benzoesäure-(4-(5-octylpyrimidin-2-yl)-phenyl)-ester
4-(5-Propylpyrimidin-2-yl)-benzoesäure-(4-(5-nonylpyrimidin-2-yl)-phenyl)-ester
4-(5-Propylpyrimidin-2-yl)-benzoesäure-(4-(5-decylpyrimidin-2-yl)-phenyl)-ester

4-(5-Pentylpyrimidin-2-yl)-benzoesäure-(4-(5-propylpyrimidin-2-yl)-phenyl)-ester
4-(5-Pentylpyrimidin-2-yl)-benzoesäure-(4-(5-pentylpyrimidin-2-yl)-phenyl)-ester
4-(5-Pentylpyrimidin-2-yl)-benzoesäure-(4-(5-hexylpyrimidin-2-yl)-phenyl)-ester
4-(5-Pentylpyrimidin-2-yl)-benzoesäure-(4-(5-heptylpyrimidin-2-yl)-phenyl)-ester
4-(5-Pentylpyrimidin-2-yl)-benzoesäure-(4-(5-octylpyrimidin-2-yl)-phenyl)-ester
4-(5-Pentylpyrimidin-2-yl)-benzoesäure-(4-(5-nonylpyrimidin-2-yl)-phenyl)-ester
4-(5-Pentylpyrimidin-2-yl)-benzoesäure-(4-(5-decylpyrimidin-2-yl)-phenyl)-ester

4-(5-Hexylpyrimidin-2-yl)-benzoesäure-(4-(5-propylpyrimidin-2-yl)-phenyl)-ester
4-(5-Hexylpyrimidin-2-yl)-benzoesäure-(4-(5-pentylpyrimidin-2-yl)-phenyl)-ester
4-(5-Hexylpyrimidin-2-yl)-benzoesäure-(4-(5-hexylpyrimidin-2-yl)-phenyl)-ester
4-(5-Hexylpyrimidin-2-yl)-benzoesäure-(4-(5-heptylpyrimidin-2-yl)-phenyl)-ester
4-(5-Hexylpyrimidin-2-yl)-benzoesäure-(4-(5-octylpyrimidin-2-yl)-phenyl)-ester
4-(5-Hexylpyrimidin-2-yl)-benzoesäure-(4-(5-nonylpyrimidin-2-yl)-phenyl)-ester
4-(5-Hexylpyrimidin-2-yl)-benzoesäure-(4-(5-decylpyrimidin-2-yl)-phenyl)-ester

4-(5-Heptylpyrimidin-2-yl)-benzoesäure-(4-(5-propylpyrimidin-2-yl)-phenyl)-ester
4-(5-Heptylpyrimidin-2-yl)-benzoesäure-(4-(5-pentylpyrimidin-2-yl)-phenyl)-ester
4-(5-Heptylpyrimidin-2-yl)-benzoesäure-(4-(5-hexylpyrimidin-2-yl)-phenyl)-ester
4-(5-Heptylpyrimidin-2-yl)-benzoesäure-(4-(5-heptylpyrimidin-2-yl)-phenyl)-ester
4-(5-Heptylpyrimidin-2-yl)-benzoesäure-(4-(5-octylpyrimidin-2-yl)-phenyl)-ester
4-(5-Heptylpyrimidin-2-yl)-benzoesäure-(4-(5-nonylpyrimidin-2-yl)-phenyl)-ester
4-(5-Heptylpyrimidin-2-yl)-benzoesäure-(4-(5-decylpyrimidin-2-yl)-phenyl)-ester

4-(5-Octylpyrimidin-2-yl)-benzoesäure-(4-(5-propylpyrimidin-2-yl)-phenyl)-ester
4-(5-Octylpyrimidin-2-yl)-benzoesäure-(4-(5-pentylpyrimidin-2-yl)-phenyl)-ester
4-(5-Octylpyrimidin-2-yl)-benzoesäure-(4-(5-hexylpyrimidin-2-yl)-phenyl)-ester
4-(5-Octylpyrimidin-2-yl)-benzoesäure-(4-(5-heptylpyrimidin-2-yl)-phenyl)-ester
4-(5-Octylpyrimidin-2-yl)-benzoesäure-(4-(5-octylpyrimidin-2-yl)-phenyl)-ester
4-(5-Octylpyrimidin-2-yl)-benzoesäure-(4-(5-nonylpyrimidin-2-yl)-phenyl)-ester
4-(5-Octylpyrimidin-2-yl)-benzoesäure-(4-(5-decylpyrimidin-2-yl)-phenyl)-ester

4-(5-Nonylpyrimidin-2-yl)-benzoesäure-(4-(5-propylpyrimidin-2-yl)-phenyl)-ester
4-(5-Nonylpyrimidin-2-yl)-benzoesäure-(4-(5-pentylpyrimidin-2-yl)-phenyl)-ester
4-(5-Nonylpyrimidin-2-yl)-benzoesäure-(4-(5-hexylpyrimidin-2-yl)-phenyl)-ester
4-(5-Nonylpyrimidin-2-yl)-benzoesäure-(4-(5-heptylpyrimidin-2-yl)-phenyl)-ester
4-(5-Nonylpyrimidin-2-yl)-benzoesäure-(4-(5-octylpyrimidin-2-yl)-phenyl)-ester
4-(5-Nonylpyrimidin-2-yl)-benzoesäure-(4-(5-nonylpyrimidin-2-yl)-phenyl)-ester
4-(5-Nonylpyrimidin-2-yl)-benzoesäure-(4-(5-decylpyrimidin-2-yl)-phenyl)-ester

4-(5-Decylpyrimidin-2-yl)-benzoesäure-(4-(5-propylpyrimidin-2-yl)-phenyl)-ester
4-(5-Decylpyrimidin-2-yl)-benzoesäure-(4-(5-pentylpyrimidin-2-yl)-phenyl)-ester
4-(5-Decylpyrimidin-2-yl)-benzoesäure-(4-(5-hexylpyrimidin-2-yl)-phenyl)-ester
4-(5-Decylpyrimidin-2-yl)-benzoesäure-(4-(5-heptylpyrimidin-2-yl)-phenyl)-ester
4-(5-Decylpyrimidin-2-yl)-benzoesäure-(4-(5-octylpyrimidin-2-yl)-phenyl)-ester
4-(5-Decylpyrimidin-2-yl)-benzoesäure-(4-(5-nonylpyrimidin-2-yl)-phenyl)-ester
4-(5-Decylpyrimidin-2-yl)-benzoesäure-(4-(5-decylpyrimidin-2-yl)-phenyl)-ester

## Beispiel 21

Eine flüssigkristalline Phase bestehend aus

11 % p-(5-Heptylpyrimidin-2-yl)-phenyl-(p-pentylbenzylether),
15 % p-(5-Heptylpyrimidin-2-yl)-phenyl-(p-hexylbenzylether),
18 % p-(5-Nonylpyrimidin-2-yl)-phenyl-(p-pentylbenzylether),
14 % p-(5-Nonylpyrimidin-2-yl)-phenyl-(p-heptylbenzylether),
23 % 2-p-Nonyloxyphenyl-5-nonylpyrimidin,
5 % p-Hexyloxybenzoesäure-4-(5-heptylpyrimidin-2-yl)-phenylester,
4 % p-Octyloxy-m-fluor-benzoesäure-4-(5-octylpyrimidin-2-yl)-phenylester und
10 % R-4-(5-Hexylpyrimidin-2-yl)-phenyl-2-chlorpropionat

zeigt K 4° $S_c$* 76° Ch 95° I.

Beispiel 22

Eine flüssigkristalline Phase bestehend aus

3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyrimidin,
15 % 2-p-Nonyloxyphenyl-5-nonylpyrimidin,
16 % 1-(p-Hexylphenyl)-2-(p-(5-heptylpyrimidin-2-yl)-phenyl)-ethan,
19 % 1-(p-Octyl-m-fluorphenyl)-2-(p-(5-octylpyrimidin-2-yl)-phenyl)-ethan,
15 % optisch aktives 3-Chlor-4-(2-octyloxy)-benzoesäure-4-(5-nonylpyrimidin-2-yl)-phenylester und
17 % optisch aktives 1-p-(2-Octyloxycarbonyl)-phenyl-2-(p-(5-heptylpyrimidin-2-yl)-phenyl)-ethan

zeigt K 9° $S_c$* 74° Ch 89° I.

Beispiel 23

Eine flüssigkristalline Phase bestehend aus

4 % 2-p-Heptyloxyphenyl-5-octylpyrimidin,
4 % 2-p-Octyloxyphenyl-5-octylpyrimidin,
4 % 2-p-Nonyloxyphenyl-5-octylpyrimidin,
16 % p-(5-Nonylpyrimidin-2-yl)-phenyl-p-pentylbenzylether,
11 % p-(5-Nonylpyrimidin-2-yl)-phenyl-p-heptylbenzylether,
13 % p-Octyloxybenzoesäure-5-(p-octylphenyl)-pyrimidin-2-ylester,
16 % p-Hexyloxybenzoesäure-5-(p-heptylphenyl)-pyrimidin-2-ylester,
11 % p-Heptyloxy-m-fluorbenzoesäure-5-(p-octylphenyl)-pyrimidin-2-ylester und
21 % 2-p-Nonyloxyphenyl-5-nonylpyrimidin

zeigt K 5° $S_c$.

Beispiel 24

Eine flüssigkristalline Phase bestehend aus

11 % p-(5-Heptylpyrimidin-2-yl)-phenyl-(p-pentylbenzylether),
9 % p-(5-Heptylpyrimidin-2-yl)-phenyl-(p-hexylbenzylether),
12 % p-(5-Nonylpyrimidin-2-yl)-phenyl-(p-pentylbenzylether),
10 % p-(5-Nonylpyrimidin-2-yl)-phenyl-(p-heptylbenzylether),
20 % 2-p-Nonyloxyphenyl-5-nonylpyrimidin,
9 % 1-(trans-4-Heptylcyclohexyl)-2-(p-(5-octylpyrimidin-2-yl)-phenyl)-ethan,
8 % 1-(trans-4-Octylcyclohexyl)-2-(p-(5-nonylpyrimidin-2-yl)-phenyl)-ethan,
10 % optisch aktives 1-(p-(2-Octyloxy-carbonyl)-phenyl)-2-(p-(5-heptylpyrimidin-2-yl)-phenyl)-ethan und
11 % 1-(p-Heptyloxy-m-fluorphenyl)-2-(p-(5-heptylpyrimidin-2-yl)-phenyl)-ethan

zeigt K 3° $S_c$* 72° Ch 93° I.

Beispiel 25

Eine flüssigkristalline Phase bestehend aus

3 % 2-p-Heptyloxyphenyl-5-heptylpyrimidin,
3 % 2-p-Octyloxyphenyl-5-heptylpyrimidin,
7 % p-(5-Heptylpyrimidin-2-yl)-phenyl-(p-pentylbenzylether),
5 % p-(5-Heptylpyrimidin-2-yl)-phenyl-(p-hexylbenzylether),
20 % 2-p-Nonyloxyphenyl-5-nonylpyrimidin,
10 % R-4-(5-Hexylpyrimidin-2-yl)-phenyl-2-chlorpropionat,
8 % 1-(p-Hexylphenyl)-2-(p-(5-heptylpyrimidin-2-yl)-phenyl)-ethan,
13 % 1-(p-Octylphenyl)-2-(p-(5-heptylpyrimidin-2-yl)-phenyl)-ethan,
11 % 1-(p-Heptylphenyl)-2-(p-(5-nonylpyrimidin-2-yl)-phenyl)-ethan und
9 % 1-(p-Octylphenyl)-2-(p-(5-nonylpyrimidin-2-yl)-phenyl)-ethan

zeigt K 8° $S_c$* 74° Ch 88° I.

Beispiel 26

Eine flüssigkristalline Phase bestehend aus

3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyrimidin,
24 % 2-p-Nonyloxyphenyl-5-nonylpyrimidin,
20 % p-Hexylbenzoesäure-5-(p-nonylphenyl)-pyrimidin-2-ylester,
10 % p-Heptylbenzoesäure-5-(p-nonylphenyl)-pyrimidin-2-ylester,
10 % p-(5-Heptylpyrimidin-2-yl)-phenyl-(p-hexylbenzyl-ether),
8 % p-(5-Nonylpyrimidin-2-yl)-phenyl-(p-heptylbenzyl-ether) und
10 % optisch aktives Ethyl-2-[p-(5-nonylpyrimidin-2-yl)-phenoxy]-propanoat

zeigt K — 8° $S_c$* 71° Ch 88° I und eine spontane Polarisation $P_s$ von 8 $nC/cm^2$ bei 20°.

## Patentansprüche

1. Verwendung von Verbindungen der Formel I,

$$R^1-A^1-Z^1-A^2-[Z^2-A^3]_m-R^2 \qquad I$$

worin
$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O und/oder —C=CH— ersetzt sein können, einer der Reste $R^1$ und $R^2$ auch H, F, Cl, Br oder CN,
$A^1$ —Pyr—Phe—, —Phe—Pyr—, —Pyr—Cy— oder —Cy—Pyr—,
Pyr Pyrimidin-2,5-diyl, Pyridin-2,5-diyl, Pyrazin-2,5-diyl oder Pyridazin-3,6-diyl,
Phe unsubstituiertes oder durch ein oder zwei F- und/oder Cl-Atome und/oder $CH_3$- und/oder CN-Gruppen substituiertes 1,4-Phenylen,
Cy trans-1,4-Cyclohexylen,
$A^2$ und $A^3$ jeweils trans-1,4-Cyclohexylen oder unsubstituiertes oder durch ein oder zwei F- und/oder Cl-Atome und/oder $CH_3$- und/oder CN-Gruppen substituiertes 1,4-Phenylen, worin auch eine oder mehrere CH-Gruppen durch N ersetzt sein können,
$Z^1$ —$CH_2CH_2$—, —CO—O—, —O—CO—, —$CH_2$—O—, oder —$OCH_2$—,
$Z^2$ —CO—O—, —O—CO—, —$CH_2$—$CH_2$—, —$CH_2O$—, —O—$CH_2$— oder eine Einfachbindung, und
m 0 oder 1
bedeuten, als Komponenten smektischer flüssigkristalliner Phasen mit ferroelektrischen Eigenschaften.

2. Smektische flüssigkristalline Phase mit ferroelektrischen Eigenschaften enthaltend mindestens zwei flüssigkristallinen Komponenten, dadurch gekennzeichnet, daß mindestens eine Komponente eine Verbindung der Formel I nach Anspruch 1 ist.

3. Phase nach Anspruch 2, dadurch gekennzeichnet, daß sie mindestens eine Komponente ausgewählt aus den Verbindungen der Teilformeln Ia, Ic und Ie enthält:

$R^1$—Pyr—Phe—$Z^1$—Phe—$R^2$ Ia
$R^1$—Pyr—Phe—$Z^1$—Phe—$Z^2$—$A^3$—$R^2$ Ic
$R^1$—Phe—Pyr—$Z^1$—Phe—$R^2$ Ie

4. Phase nach Anspruch 3, dadurch gekennzeichnet, daß die Verbindungen der Formeln Ia, Ic und Ie solche der folgenden Teilformeln sind:

$R^1$—Pyr—Phe—$OCH_2$—Phe—$R^2$ Iaa
$R^1$—Pyr—Phe—$CH_2CH_2$—Phe—$R^2$ Iab
$R^1$—Pyr—Phe—$CH_2O$—Phe—$R^2$ Iac
$R^1$—Pyr—Phe—OCO—Phe—$R^2$ Iad
$R^1$—Pyr—Phe—COO—Phe—$R^2$ Iae

$R^1$—Pyr—Phe—$OCH_2$—Phe—OCO—Cy—$R^2$ Ica
$R^1$—Pyr—Phe—$OCH_2$—Phe—OCO—Phe—$R^2$ Icb
$R^1$—Pyr—Phe—$OCH_2$—Phe—Pyr—$R^2$ Icc

$R^1$—Pyr—Phe—OCO—Phe—Phe—$R^2$ Icd
$R^1$—Pyr—Phe—OCO—Phe—Pyr—$R^2$ Ice
$R^1$—Pyr—Phe—COO—Phe—Phe—$R^2$ Icf
$R^1$—Pyr—Phe—COO—Pyr—Phe—$R^2$ Icg

$R^1$—Phe—Pyr—$OCH_2$—Phe—$R^2$ Iea
$R^1$—Phe—Pyr—$CH_2CH_2$—Phe—$R^2$ Ieb
$R^1$—Phe—Pyr—$CH_2O$—Phe—$R^2$ Iec
$R^1$—Phe—Pyr—OCO—Phe—$R^2$ Ied
$R^1$—Phe—Pyr—COO—Phe—$R^2$ Iee

5. Phase nach mindestens einem der Ansprüche 2 bis 4, dadurch gekennzeichnet, daß $R^1$ und $R^2$ Alkyl, Alkoxy, Oxaalkyl, Alkanoyloxy oder Alkoxycarbonyl bedeuten.

6. Phase nach mindestens einem der Ansprüche 2 bis 5, dadurch gekennzeichnet, daß $A^1$ —Pyr—Phe— oder —Phe—Pyr— bedeutet.

7. Phase nach Anspruch 6, dadurch gekennzeichnet daß —$A^1$— ein Strukturelement ausgewählt aus der Gruppe der Formeln 1 bis 4 ist :

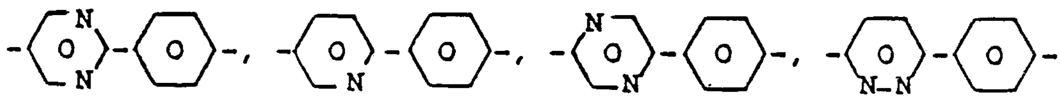

1 2 3 4

8. Phase nach Anspruch 7, dadurch gekennzeichnet, daß $A^1$ ein Strukturelement der Formel 1 oder 2 ist.

9. Phase nach mindestens einem der Ansprüche 2 bis 8, dadurch gekennzeichnet, daß $A^2$ unsubstituiertes 1,4-Phenylen oder trans-1,4-Cyclohexylen ist.

10. Phase nach mindestens einem der Ansprüche 2 bis 9, dadurch gekennzeichnet, daß $Z^1$ —$OCH_2$—, —O—CO— oder —CO—O— ist.

11. Phase nach mindestens einem der Ansprüche 2 bis 10, dadurch gekennzeichnet, daß m 0 bedeutet.

12. Phase nach Anspruch 2, dadurch gekennzeichnet, daß sie mindestens eine Komponente ausgewählt aus den Verbindungen der Formeln I1 bis I20 enthält :

R—Pyr—Phe—$OCH_2$—Phe—R' I1
R—Pyr—Phe—$CH_2O$—Phe—R' I2
R—Pyr—Phe—OCO—Phe—R' I3
R—Pyr—Phe—COO—Phe—R' I4
R—Pyr—Phe—$CH_2CH_2$—Phe—R' I5
R—Phe—Pyr—$OCH_2$—Phe—R' I6
R—Phe—Pyr—$CH_2O$—Phe—R' I7
R—Phe—Pyr—OCO—Phe—R' I8
R—Phe—Pyr—COO—Phe—R' I9
R—Phe—Pyr—$CH_2CH_2$—Phe—R' I10
R—Pyr—Phe—$OCH_2$—Cy—R' I11
R—Pyr—Phe—$CH_2O$—Cy—R' I12
R—Pyr—Phe—OCO—Cy—R' I13
R—Pyr—Phe—COO—Cy—R' I14
R—Pyr—Phe—$CH_2CH_2$—Cy—R' I15
R—Phe—Pyr—$OCH_2$—Cy—R' I16
R—Phe—Pyr—$CH_2O$—Cy—R' I17
R—Phe—Pyr—OCO—Cy—R' I18
R—Phe—Pyr—COO—Cy—R' I19
R—Phe—Pyr—$CH_2CH_2$—Cy—R' I20

worin R und R' geradkettige Alkyl-, Alkoxy-, Alkanoyloxy- oder Alkoxycarbonylgruppen mit jeweils 5 bis 12 C-Atomen bedeuten und die vorstehend genannten Formeln die beiden möglichen 2,5-Stellungsisomeren bezüglich der Pyridin-2,5- bzw. Pyrimidin-2,5-diyl-Gruppe umschließen.

13. Phase nach Anspruch 2, dadurch gekennzeichnet, daß sie mindestens einen stickstoffhaltigen heterocyclischen Ester der Formel IV enthält,

$$R^1-A^1-Z^1-A^2-[Z^2-A^3]_m-R^2 \quad (IV)$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können, einer der Reste $R^1$ und $R^2$ auch H, F, Cl, Br oder CN,

$A^1$ ein unsubstituiertes oder durch ein oder zwei F- und/oder Cl- und/oder Br-Atome und/oder $CH_3$- und/oder CN-Gruppen substituiertes Strukturelement ausgewählt aus der Gruppe der Formeln 1 bis 4 :

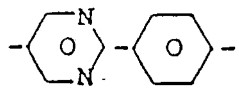
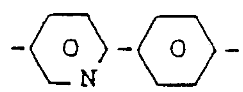
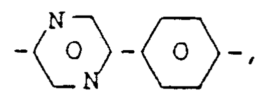

1 2 3

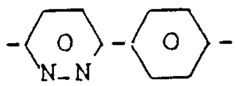

4

$A^2$ und $A^3$ jeweils trans-1,4-Cyclohexylen oder unsubstituiertes oder durch ein oder zwei F- und/oder Cl-Atome und/oder Br-Atome und/oder $CH_3$- und/oder CN-Gruppen substituiertes 1,4-Phenylen, worin auch eine oder mehrere CH-Gruppen durch N ersetzt sein können

$Z^1$ —CO—O— oder —O—CO—

$Z^2$ —CO—O—, —O—CO—, —$CH_2CH_2$—, —$CH_2O$—, —O—$CH_2$— oder eine Einfachbindung, und

m 0 oder 1

bedeuten, mit den Maßgaben, daß

(1) die Verbindung mindestens eine lateral substituierte 1,4-Phenylen-Gruppe enthält und

(2) im Falle $m = 0$ und $R^1 — A^1 — Z^1$ = Alkyl

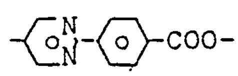

$A^2$ lateral durch Fluor substituiertes 1,4-Phenylen oder in ortho-Stellung zu $R^2$ durch Cl, Br, $CH_3$ oder CN substituiertes 1,4-Phenylen bedeutet.

14. Phase nach mindestens einem der Ansprüche 2 bis 13, dadurch gekennzeichnet, daß sie mindestens eine optisch aktive Verbindung enthält.

15. Phase nach mindestens einem der Ansprüche 2 bis 14, dadurch gekennzeichnet, daß sie andere Bestandteile ausgewählt aus den Verbindungen der folgenden Formeln enthält :

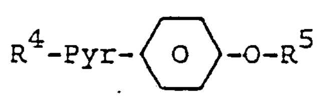
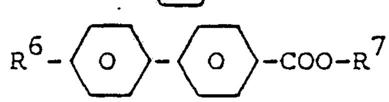
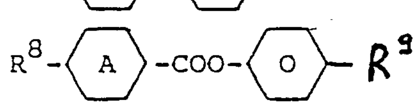

worin $R^4$ und $R^5$ jeweils unabhängig voneinander n-Alkyl mit 5 bis 12 C-Atomen bedeutet, $R^6$, $R^7$, $R^8$ und $R^9$ jeweils unabhängig voneinander geradkettige oder verzweigte, ggf. chirale, Alkyl-, Alkoxy-, Alkoxycarbonyl- oder Alkanoyloxygruppen mit 5 bis 12 C-Atomen und Ring A 1,4-Phenylen oder trans-1,4-Cyclohexylen bedeutet.

16. Phase nach Anspruch 15, dadurch gekennzeichnet, daß sie mindestens eine Komponente der Formel

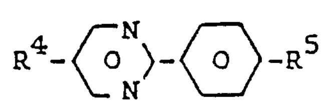

Vj

enthält, worin $R^4$ und $R^5$ jeweils geradkettiges Alkyl, Alkoxy, Alkanoyloxy der Alkoxycarbonyl mit jeweils 3 bis 12 C-Atomen ist.

17. Elektrooptisches Anzeigeelement, dadurch gekennzeichnet, daß es als Dielektrikum eine Phase nach mindestens einem der Ansprüche 2 bis 16 enthält.

18. Pyrimidinderivate der Formel II

$$R^1\text{-}A^1\text{-}Pyr\text{-}Z^1\text{-}A^2\text{-}[Z^2\text{-}A^3]_m\text{-}R^2 \qquad (II)$$

worin

$R^1$ und $R^2$ jeweils eine Alkylgruppe mit 1 bis 12 C-Atomen, worin auch eine oder zwei nicht benachbarte $CH_2$-Gruppen durch —O—, —CO—, —O—CO—, —CO—O oder —CH=CH— ersetzt sein können, einer der Reste $R^1$ und $R^2$ auch H, F, Cl, Br oder CN,

Pyr Pyrimidin-2,5-diyl,

$A^2$ und $A^3$ jeweils trans-1,4-Cyclohexylen oder unsubstituiertes oder durch ein oder zwei F- und/oder Cl-Atome und/oder $CH_3$- und/oder CN-Gruppen substituiertes 1,4-Phenylen,

$A^1$ unsubstituiertes oder durch ein oder zwei F- und/oder Cl-Atome und/oder $CH_3$- und/oder CN-Gruppen substituiertes 1,4-Phenylen,

$Z^1$ —$OCH_2$— oder —O—CO—,

$Z^2$ —CO—O—, —O—CO—, —$CH_2CH_2$—, —$CH_2O$—, —O—$CH_2$— oder eine Einfachbindung und

m 0 oder 1

bedeuten, mit der Maßgabe, daß im Falle $Z^1$ = —O—CO— $A^1$ unsubstituiertes 1,4-Phenylen und $A^2$ und $A^3$ jeweils trans-1,4-Cyclohexylen oder unsubstituiertes 1,4-Phenylen bedeuten.

19. Pyrimidinderivate nach Anspruch 18, dadurch gekennzeichnet, daß $R^1$ und $R^2$ jeweils geradkettige oder verzweigte Alkyl- oder Alkoxygruppen mit jeweils 3 bis 10 C-Atomen bedeuten.

20. Pyrimidinderivate nach Anspruch 18 oder 19, dadurch gekennzeichnet, daß m 0 und $A^2$ trans-1,4-Cyclohexylen oder unsubstituiertes 1,4-Phenylen bedeutet.

21. Pyrimidinderivate nach mindestens einem der Ansprüche 18 bis 20, dadurch gekennzeichnet, daß $A^1$ unsubstituiertes 1,4-Phenylen ist.

22. Vierkernige Verbindungen der Formel III,

$$R^1\text{-}A^1\text{-}Z^3\text{-}A^{1'}\text{-}R^2 \qquad (III)$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können,

$A^1$ und $A^{1'}$ jeweils —Pyr—Phe—, —Phe—Pyr—, —Pyr—Cyr— oder —Cy—Pyr—,

Pyr Pyrimidin-2,5-diyl,

Phe unsubstituiertes oder durch ein oder zwei F- und/oder Cl-Atome und/oder $CH_3$- und/oder CN-Gruppen substituiertes 1,4-Phenylen,

Cy trans-1,4-Cyclohexylen, und

$Z^3$ —CO—O— oder —$CH_2O$—

bedeutet.

23. Verbindungen der Formel III nach Anspruch 22, dadurch gekennzeichnet, daß $R^1$ un $R^2$ jeweils geradkettige oder verzweigte Alkyl- oder Alkoxygruppen mit jeweils 3 bis 10 C-Atomen bedeuten.

24. Verbindungen nach mindestens einem der Ansprüche 22 und 23, dadurch gekennzeichnet, daß $A^1$ und $A^{1'}$ —Pyr—Phe— oder —Phe—Pyr— bedeuten.

25. Verbindungen nach mindestens einem der Ansprüche 22 bis 24 gekennzeichnet durch die Teilformeln IIIa bis IIId :

$R^1$—Pyr—Phe—COO—Phe—Pyr—$R^2$ IIIa
$R^1$—Pyr—Phe—COO—Pyr—Phe—$R^2$ IIIb
$R^1$—Pyr—Phe—$CH_2O$—Phe—Pyr—$R^2$ IIIc
$R^1$—Pyr—Phe—$CH_2O$—Pyr—Phe—$R^2$ IIId

**Claims**

1. Use of compounds of the formula I

$$R^1\text{-}A^1\text{-}Z^1\text{-}A^2\text{-}[Z^2\text{-}A^3]_m\text{-}R^2 \qquad (I)$$

in which

$R^1$ and $R^2$ are in each case an alkyl group, having 1 to 12 C atoms, in which one or more non-

neighbouring $CH_2$ groups may also be replaced by —O—, —CO—, —O—CO—, —O—COO—, —CO—O— and/or —CH=CH—, or one of the radicals $R^1$ and $R^2$ is also H, F, Cl, Br or CN,

$A_1$ is —Pyr—Phe—, —Phe—Pyr—, —Pyr—Cy— or —Cy—Pyr—,

Pyr is pyrimidin-2,5-diyl, pyridin-2,5-diyl, pyrazin-2,5-diyl or pyridazin-3,6-diyl,

Phe is 1,4-phenylene which is unsubstituted or substituted by one or two F and/or Cl atoms and/or $CH_3$ and/or CN groups,

Cy is trans-1,4-cyclohexylene,

$A^2$ and $A^3$ are in each case trans-1,4-cyclohexylene, or 1,4-phenylene, which is unsubstituted or substituted by one or two F and/or Cl atoms and/or $CH_3$ and/or CN groups, in which one or more CH groups may also be replaced by N,

$Z^1$ is —$CH_2CH_2$—, —CO—O—, —O—CO—, —$CH_2$—O— or —$OCH_2$—,

$Z^2$ is —CO—O—, —O—CO—, —$CH_2CH_2$—, —$CH_2O$—, —O—$CH_2$— or a single bond, and

m is 0 or 1,

as components of smectic liquid-crystalline phases having ferroelectric properties.

2. Smectic liquid-crystalline phase having ferroelectric properties, containing at least two liquid-crystalline components, characterized in that at least one component is a compound of the formula I according to Claim 1.

3. Phase according to Claim 2, characterized in that it contains at least one component selected from the compounds of the subformulae Ia, Ic and Ie :

$R^1$—Pyr—Phe—$Z^1$—Phe—$R^2$ Ia
$R^1$—Pyr—Phe—$Z^1$—Phe—$Z^2$—$A^3$—$R^2$ Ic
$R^1$—Phe—Pyr—$Z^1$—Phe—$R^2$ Ie

4. Phase according to Claim 3, characterized in that the compounds of the formulae Ia, Ic and Ie are those of the following subformulae :

$R^1$—Pyr—Phe—$OCH_2$—Phe—$R^2$ Iaa
$R^1$—Pyr—Phe—$CH_2CH_2$—Phe—$R^2$ Iab
$R^1$—Pyr—Phe—$CH_2O$—Phe—$R^2$ Iac
$R^1$—Pyr—Phe—OCO—Phe—$R^2$ Iad
$R^1$—Pyr—Phe—COO—Phe—$R^2$ Iae

$R^1$—Pyr—Phe—$OCH_2$—Phe—OCO—Cy—$R^2$ Ica
$R^1$—Pyr—Phe—$OCH_2$—Phe—OCO—Phe—$R^2$ Icb
$R^1$—Pyr—Phe—$OCH_2$—Phe—Pyr—$R^2$ Icc
$R^1$—Pyr—Phe—OCO—Phe—Phe—$R^2$ Icd
$R^1$—Pyr—Phe—OCO—Phe—Pyr—$R^2$ Ice
$R^1$—Pyr—Phe—COO—Phe—Phe—$R^2$ Icf
$R^1$—Pyr—Phe—COO—Pyr—Phe—$R^2$ Icg

$R^1$—Phe—Pyr—$OCH_2$—Phe—$R^2$ Iea
$R^1$—Phe—Pyr—$CH_2CH_2$—Phe—$R^2$ Ieb
$R^1$—Phe—Pyr—$CH_2O$—Phe—$R^2$ Iec
$R^1$—Phe—Pyr—OCO—Phe—$R^2$ Ied
$R^1$—Phe—Pyr—COO—Phe—$R^2$ Iee

5. Phase according to at least one of Claims 2 to 4, characterized in that $R^1$ and $R^2$ are alkyl, alkoxy, oxaalkyl, alkanoyloxy or alkoxycarbonyl.

6. Phase according to at least one of Claims 2 to 5, characterized in that $A^1$ is —Pyr—Phe— or —Phe—Pyr—.

7. Phase according to Claim 6, characterized in that —$A^1$— is a structural element selected from the group comprising the formulae 1 to 4 :

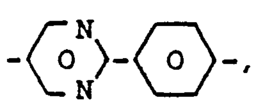, 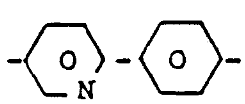, 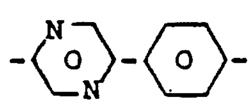, 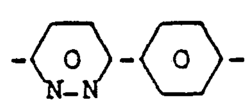

1 2 3 4

8. Phase according to Claim 7, characterized in that $A^1$ is a structural element of the formula 1 or 2.

9. Phase according to at least one of Claims 2 to 8, characterized in that $A^2$ is unsubstituted 1,4-phenylene or trans-1,4-cyclohexylene.

10. Phase according to at least one of Claims 2 to 9, characterized in that $Z^1$ is —$OCH_2$—, —O—CO— or —CO—O—.

11. Phase according to at least one of Claims 2 to 10, characterized in that m is 0.

12. Phase according to Claim 2, characterized in that it contains at least one component selected from the compounds of the formulae I1 to I20 :

| | |
|---|---|
| R—Pyr—Phe—$OCH_2$—Phe—R' | I1 |
| R—Pyr—Phe—$CH_2O$—Phe—R' | I2 |
| R—Pyr—Phe—OCO—Phe—R' | I3 |
| R—Pyr—Phe—COO—Phe—R' | I4 |
| R—Pyr—Phe—$CH_2CH_2$—Phe—R' | I5 |
| R—Phe—Pyr—$OCH_2$—Phe—R' | I6 |
| R—Phe—Pyr—$CH_2O$—Phe—R' | I7 |
| R—Phe—Pyr—OCO—Phe—R' | I8 |
| R—Phe—Pyr—COO—Phe—R' | I9 |
| R—Phe—Pyr—$CH_2CH_2$—Phe—R' | I10 |
| R—Pyr—Phe—$OCH_2$—Cy—R' | I11 |
| R—Pyr—Phe—$CH_2O$—Cy—R' | I12 |
| R—Pyr—Phe—OCO—Cy—R' | I13 |
| R—Pyr—Phe—COO—Cy—R' | I14 |
| R—Pyr—Phe—$CH_2CH_2$—Cy—R' | I15 |
| R—Phe—Pyr—$OCH_2$—Cy—R' | I16 |
| R—Phe—Pyr—$CH_2O$—Cy—R' | I17 |
| R—Phe—Pyr—OCO—Cy—R' | I18 |
| R—Phe—Pyr—COO—Cy—R' | I19 |
| R—Phe—Pyr—$CH_2CH_2$—Cy—R' | I20 |

in which R and R' are straight-chain alkyl, alkoxy, alkanoyloxy or alkoxycarbonyl groups having 5 to 12 C atoms in each case and the abovementioned formulae include the two possible 2,5-positional isomers with respect to the pyridin-2,5-diyl or pyrimidin-2,5-diyl group.

13. Phase according to Claim 2, characterized in that it contains at least one nitrogen-containing heterocyclic ester of the formula IV

$$R^1-A^1-Z^1-A^2-[Z^2-A^3]_m-R^2 \qquad (IV)$$

in which

$R^1$ and $R^2$ are in each case an alkyl group, having 1 to 12 C atoms, in which one or more non-neighbouring $CH_2$ groups may also be replaced by —O—, —CO—, —O—CO—, —O—COO—, —CO—O— and/or —CH=CH—, and one of the radicals $R^1$ and $R^2$ is also H, F, Cl, Br or CN,

$A^1$ is a structural element selected from the group comprising the formulae 1 to 4 :

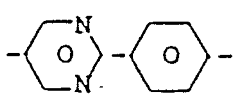
1

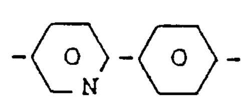
2

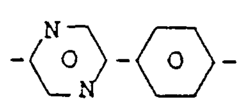
3

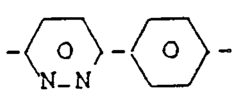
4

which is unsubstituted or substituted by one or two F and/or Cl and/or Br atoms and/or $CH_3$ and/or CN groups,

$A^2$ and $A^3$ are in each case trans-1,4-cyclohexylene, or 1,4-phenylene, which is unsubstituted or substituted by one or two F and/or Cl atoms and/or Br atoms and/or $CH_3$ and/or CN groups, in which one or more CH groups may also be replaced by N,

$Z^1$ is —CO—O— or —O—CO—

$Z^2$ is —CO—O—, —O—CO—, —$CH_2CH_2$—, —$CH_2O$—, —O—$CH_2$— or a single bond, and

m is 0 or 1,

with the proviso that

(1) the compound contains at least one laterally substituted 1,4-phenylene group and

(2) in the case of m = 0 and $R^1$ — $A^1$ — $Z^1$ = Alkyl

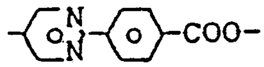

$A^2$ is 1,4-phenylene laterally substituted by fluorine, or 1,4-phenylene substituted in the ortho position with respect to $R^2$ by Cl, Br, $CH_3$ or CN.

14. Phase according to at least one of Claims 2 to 13, characterized in that it contains at least one optically active compound.

15. Phase according to at least one of Claims 2 to 14, characterized in that it contains other components selected from the compounds of the following formulae :

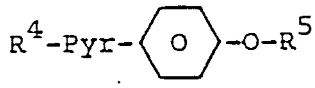

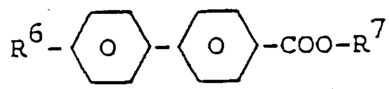

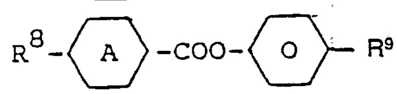

in which $R^4$ and $R^5$, in each case independently of one another, are n-alkyl having 5 to 12 C atoms, and $R^6$, $R^7$, $R^8$ and $R^9$, in each case independently of one another, are straight-chain or branched, optionally chiral alkyl, alkoxy, alkoxycarbonyl or alkanoyloxy groups having 5 to 12 C atoms and ring A is 1,4-phenylene or trans-1,4-cyclohexylene.

16. Phase according to Claim 15, characterized in that it contains at least one component of the formula

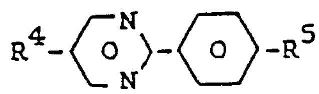 Vj

in which $R^4$ and $R^5$ are in each case straight-chain alkyl, alkoxy, alkanoyloxy or alkoxycarbonyl having 3 to 12 C atoms in each case.

17. Electro-optical display element, characterized in that it contains a phase according to at least one of Claims 2 to 15 as dielectric.

18. Pyrimidine derivatives of the formula II

$$R^1\text{-}A^1\text{-}Pyr\text{-}Z^1\text{-}A^2\text{-}[Z^2\text{-}A^3]_m\text{-}R^2 \qquad (II)$$

in which

$R^1$ and $R^2$ are in each case an alkyl group, having 1 to 12 C atoms, in which one or two non-neighbouring $CH_2$ groups may also be replaced by —O—, —CO—, —O—CO—, —CO—O— or —CH=CH—, and one of the radicals $R^1$ and $R^2$ is also H, F, Cl, Br or CN,

Pyr is pyrimidin-2,5-diyl,

$A^2$ and $A^3$ are in each case trans-1,4-cyclohexylene, or 1,4-phenylene which is unsubstituted or substituted by one or two F and/or Cl atoms and/or $CH_3$ and/or CN groups,

$A^1$ is 1,4-phenylene which is unsubstituted or substituted by one or two F and/or Cl atoms and/or $CH_3$ and/or CN groups,

$Z^1$ is —$OCH_2$— or —O—CO—,

$Z^2$ is —CO—O, —O—CO—, —$CH_2CH_2$—, —$CH_2O$—, —O—$CH_2$— or a single bond, and

m is 0 or 1,

with the proviso that, in the case of $Z^1$ = —O—CO—, $A^1$ is unsubstituted 1,4-phenylene and $A^2$ and $A^3$ are in each case trans-1,4-cyclohexylene or unsubstituted 1,4-phenylene.

19. Pyrimidine derivatives according to Claim 18, characterized in that $R^1$ and $r^2$ are in each case straight-chain or branched alkyl or alkoxy groups having 3 to 10 C atoms in each case.

20. Pyrimidine derivatives according to Claim 18 or 19, characterized in that m is 0 and $A^2$ is trans-1,4-cyclohexylene or unsubstituted 1,4-phenylene.

21. Pyrimidine derivatives according to at least one of Claims 18 to 20, characterized in that $A^1$ is unsubstituted 1,4-phenylene.

22. Tetranuclear compounds of the formula III

$$R^1-A^1-Z^3-A^{1'}-R^2 \qquad (III)$$

in which

$R^1$ and $R^2$ are in each case an alkyl group, having 1 to 12 C atoms, in which one or more non-neighbouring $CH_2$ groups may also be replaced by —O—, —CO—, —O—CO—, —O—COO—, —CO—O— and/or —CH=CH—,

$A^1$ and $A^{1'}$ are in each case —Pyr—Phe—, —Phe—Pyr—, —Pyr—Cy— or —Cy—Pyr—,

Pyr is a pyrimidin-2,5-diyl,

Phe is 1,4-phenylene which is unsubstituted or substituted by one or two F and/or Cl atoms and/or $CH_3$ and/or CN groups,

Cy is trans-1,4-cyclohexylene and

$Z^3$ is —CO—O or —$CH_2$O—.

23. Compounds of the formula III according to Claim 22, characterized in that $R^1$ and $R^2$ are in each case straight-chain or branched alkyl or alkoxy groups having 3 to 10 C atoms in each case.

24. Compounds according to at least one of Claims 22 and 23, characterized in that $A^1$ and $A^{1'}$ are —Pyr—Phe— or —Phe—Pyr—.

25. Compounds according to at least one of Claims 22 to 24, characterized by the subformulae IIIa to IIId :

$R^1$—Pyr—Phe—COO—Phe—Pyr—$R^2$ IIIa
$R^1$—Pyr—Phe—COO—Pyr—Phe—$R^2$ IIIb
$R^1$—Pyr—Phe—$CH_2$O—Phe—Pyr—$R^2$ IIIc
$R^1$—Pyr—Phe—$CH_2$O—Pyr—Phe—$R^2$ IIId

## Revendications

1. Application de composés de formule I,

$$R_1-A^1-Z^1-A^2-[Z^2-A^3]_m-R^2 \qquad I$$

où

$R^1$ et $R^2$ représentent chacun un groupe alcoyle en $C_1$ à $C_{12}$, où également un ou plusieurs groupes $CH_2$ non voisins peuvent être remplacés par —O—, —CO—, —O—CO—, —O—COO—, —CO—O— et/ou —CH=CH—, l'un des radicaux $R^1$ et $R^2$ représente également H, F, Cl, Br ou CN,

$A^1$ représente —Pyr—Phe—, —Phe—Pyr—, —Pyr—Cy— ou —Cy—Pyr—,

Pyr représente pyrimidin-2,5-diyle, pyridin-2,5-diyle, pyrazin-2,5-diyle ou pyridazin-3,6-diyle,

Phe représente un 1,4-phénylène non substitué ou substitué par 1 ou 2 atomes de F et/ou Cl et/ou groupes $CH_3$ et/ou CN,

Cy représente un trans-1,4-cyclohexylène,

$A^2$ et $A^3$ représentent chacun un trans-1,4-cyclohexylène ou un 1,4-phénylène non substitué ou substitué par 1 ou 2 atomes de F et/ou Cl et/ou groupes $CH_3$ et/ou CN, où également un ou plusieurs groupes CH peuvent être remplacés par N,

$Z^1$ représente —$CH_2CH_2$—, —CO—O—, —O—CO—, —$CH_2$—O—, ou —O$CH_2$—,

$Z^2$ représente —CO—O—, —O—CO—, —$CH_2CH_2$—, —$CH_2$O—, —O—$CH_2$— ou une liaison simple et m vaut 0 ou 1,

comme composants de phases cristallines smectiques à propriétés ferro-électriques.

2. Phase cristalline liquide smectique à propriétés ferro-électriques contenant au moins deux composants cristallins liquides, caractérisée en ce qu'au moins un composant est un composé de formule I selon la revendication 1.

3. Phase selon la revendication 2, caractérisée en ce qu'elle contient au moins un composant choisi parmi les composés de formules partielles Ia, Ic et Ie :

$R^1$—Pyr—Phe—$Z^1$—Phe—$R^2$ Ia
$R^1$—Pyr—Phe—$Z^1$—Phe—$Z^2$—$A^3$—$R^2$ Ic
$R^1$—Phe—Pyr—$Z^1$—Phe—$R^2$ Ie

4. Phase selon la revendication 3, caractérisée en ce que les composés de formules Ia, Ic et Ie sont ceux de formules partielles ci-dessous :

$R^1$—Pyr—Phe—O$CH_2$—Phe—$R^2$ Iaa
$R^1$—Pyr—Phe—$CH_2CH_2$—Phe—$R^2$ Iab
$R^1$—Pyr—Phe—$CH_2$O—Phe—$R^2$ Iac
$R^1$—Pyr—Phe—OCO—Phe—$R^2$ Iad
$R^1$—Pyr—Phe—COO—Phe—$R^2$ Iae

$R^1$—Pyr—Phe—$OCH_2$—Phe—OCO—Cy—$R^2$ Ica
$R^1$—Pyr—Phe—$OCH_2$—Phe—OCO—Phe—$R^2$ Icb
$R^1$—Pyr—Phe—$OCH_2$—Phe—Pyr—$R^2$ Icc
$R^1$—Pyr—Phe—OCO—Phe—Phe—$R^2$ Icd
$R^1$—Pyr—Phe—OCO—Phe—Pyr—$R^2$ Ice
$R^1$—Pyr—Phe—COO—Phe—Phe—$R^2$ Icf
$R^1$—Pyr—Phe—COO—Pyr—Phe—$R^2$ Icg

$R^1$—Phe—Pyr—$OCH_2$—Phe—$R^2$ Iea
$R^1$—Phe—Pyr—$CH_2CH_2$—Phe—$R^2$ Ieb
$R^1$—Phe—Pyr—$CH_2O$—Phe—$R^2$ Iec
$R^1$—Phe—Pyr—OCO—Phe—$R^2$ Ied
$R^1$—Phe—Pyr—COO—Phe—$R^2$ Iee

5. Phase selon au moins l'une des revendications 2 à 4, caractérisée en ce que $R^1$ et $R^2$ représentent un alcoyle, alkoxy, oxaalcoyle, alcanoyloxy ou alkoxycarbonyle.

6. Phase selon au moins l'une des revendications 2 à 5, caractérisée en ce que $A^1$ représente un —Pyr—Phe— ou —Phe—Pyr—.

7. Phase selon la revendication 6, caractérisée en ce que —$A^1$— est un élément de structure choisi dans le groupe de formules 1 à 4 :

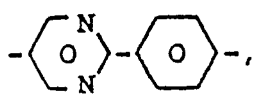
1

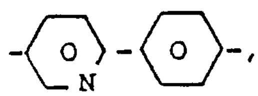
2

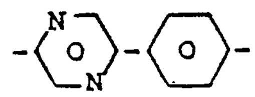
3

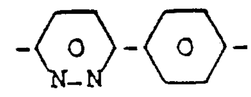
4

8. Phase selon la revendication 7, caractérisée en ce que $A^1$ est un élément de structure de formule 1 ou 2.

9. Phase selon au moins l'une des revendications 2 à 8, caractérisée en ce que $A^2$ est un 1,4-phénylène ou trans-1,4-cyclohexylène non substitué.

10. Phase selon au moins l'une des revendications 2 à 9, caractérisée en ce que $Z^1$ représente —$OCH_2$—, —O—CO— ou —CO—O—.

11. Phase selon au moins l'une des revendications 2 à 10, caractérisée en ce que m vaut 0.

12. Phase selon la revendication 2, caractérisée en ce qu'elle contient au moins un composant choisi parmi les composés de formules I1 à I20 :

R—Pyr—Phe—$OCH_2$—Phe—R' I1
R—Pyr—Phe—$CH_2O$—Phe—R' I2
R—Pyr—Phe—OCO—Phe—R' I3
R—Pyr—Phe—COO—Phe—R' I4
R—Pyr—Phe—$CH_2CH_2$—Phe—R' I5
R—Phe—Pyr—$OCH_2$—Phe—R' I6
R—Phe—Pyr—$CH_2O$—Phe—R' I7
R—Phe—Pyr—OCO—Phe—R' I8
R—Phe—Pyr—COO—Phe—R' I9
R—Phe—Pyr—$CH_2CH_2$—Phe—R' I10
R—Pyr—Phe—$OCH_2$—Cy—R' I11
R—Pyr—Phe—$CH_2O$—Cy—R' I12
R—Pyr—Phe—OCO—Cy—R' I13
R—Pyr—Phe—COO—Cy—R' I14
R—Pyr—Phe—$CH_2CH_2$—Cy—R' I15
R—Phe—Pyr—$OCH_2$—Cy—R' I16
R—Phe—Pyr—$CH_2O$—Cy—R' I17
R—Phe—Pyr—OCO—Cy—R' I18
R—Phe—Pyr—COO—Cy—R' I19
R—Phe—Pyr—$CH_2CH_2$—Cy—R' I20

où R et R' représentent des groupes alcoyles, alkoxy, alcanoyloxy ou alkoxycarbonyles à chaîne droite avec à chaque fois de 5 à 12 atomes de carbone et les formules mentionnées ci-dessus comprennent les deux isomères possibles en position 2,5 par rapport au groupe pyridin-2,5 ou pyrimidin-2,5-diyle.

13. Phase selon la revendication 2, caractérisée en ce qu'elle contient au moins un ester éthérocyclique contenant de l'azote de formule IV

$$R^1-A^1-Z^1-A^2-[Z^2-A^3]_m-R^2 \qquad IV$$

où

$R^1$ et $R^2$ représentent chacun un groupe alcoyle en $C_1$ à $C_{12}$, où également un ou plusieurs groupes $CH_2$ non voisins peuvent être remplacés par —O—, —CO—, —O—CO—, —O—COO—, —CO—O— et/ou —CH=CH—, l'un des radicaux $R^1$ et $R^2$ représente également H, F, Cl, Br ou CN.

$A^1$ représente un élément de structure non substitué ou substitué par un ou deux atomes de F et/ou Cl et/ou Br et/ou groupes $CH_3$ et/ou CN choisis dans le groupe de formules 1 à 4 :

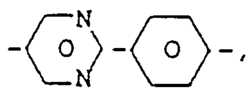
1

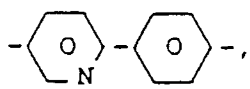
2

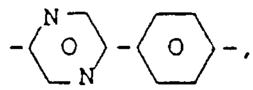
3

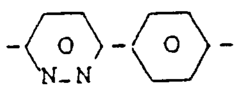
4

$A^2$ et $A^3$ représentent chacun un trans-1,4-cyclohexylène ou un 1,4-phénylène non substitué ou substitué par 1 ou 2 atomes de F et/ou Cl et/ou Br et/ou groupes $CH_3$ et/ou CN, où également un ou plusieurs groupes CH peuvent être remplacés par N,

$Z^1$ représente —CO—O— ou —O—CO—,

$Z^2$ représente —CO—O—, —O—CO—, —$CH_2CH_2$—, —$CH_2O$—, —O—$CH_2$— ou une liaison simple, et

m vaut 0 ou 1

avec ces précisions que

(1) le composé contient au moins un groupe 1,4-phénylène à substitution latérale et

(2) au cas où m = 0 et $R^1 — A^1 — Z^1$ = alcoyl

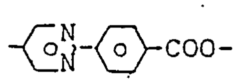

$A^2$ représente un 1,4-phénylène substitué latéralement par un fluor ou un 1,4-phénylène substitué en position ortho par rapport à $R^2$ par Cl, Br, $CH_3$ ou CN.

14. Phase selon au moins l'une des revendications 2 à 13, caractérisée en ce qu'elle contient au moins un composé optiquement actif.

15. Phase selon au moins l'une des revendications 2 à 14, caractérisée en ce qu'elle contient d'autres composés choisis parmi les composés de formules suivantes :

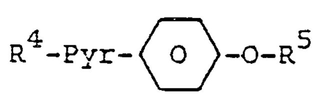

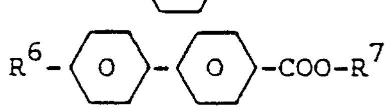

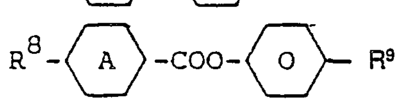

où $R^4$ et $R^5$ représentent chacun indépendamment l'un de l'autre un n-alcoyle en $C_5$ à $C_{12}$, $R^6$, $R^7$, $R^8$ et $R^9$ représentent chacun indépendamment l'un de l'autre des groupes alcoyles, alkoxy, alkoxycarbonyles ou alcanoyloxy à chaîne droite ou ramifiée, éventuellement chiral en $C_5$ à $C_{12}$ et le noyau A représente un 1,4-phénylène ou un trans-1,4-cyclohexylène.

16. Phase selon la revendication 15, caractérisée en ce qu'elle contient au moins un composant de formule

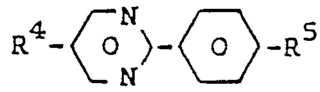

Vj

où $R^4$ et $R^5$ représentent chacun un alcoyle, alkoxy, alcanoyloxy ou alkoxycarbonyle à chaîne droite avec à chaque fois de 3 à 12 atomes de carbone.

17. Elément d'affichage électro-optique, caractérisé en ce qu'il contient comme diélectrique une phase selon au moins une des revendications 2 à 16.

18. Dérivés de pyrimidine de formule II

$$R^1-A^1-Pyr-Z^1-A^2-[Z^2-A^3]_m-R^2 \qquad (II)$$

où

$R^1$ et $R^2$ représentent chacun un groupe alcoyle en $C_1$ à $C_{12}$, où également un ou deux groupes $CH_2$ non voisins peuvent être remplacés par —O—, —CO—, —O—CO—, —CO—O— ou —CH=CH—, l'un des radicaux $R^1$ et $R^2$ représente également H, F, Cl, Br ou CN

Pyr représente un pyrimidin-2,5-diyle,

$A^2$ et $A^3$ représentent chacun un trans-1,4-cyclo-hexylène ou un 1,4-phénylène non substitué ou substitué par 1 ou 2 atomes de F et/ou Cl et/ou groupes $CH_3$ et/ou CN,

$A^1$ représente un 1,4-phénylène non substitué ou substitué par 1 ou 2 atomes de F et/ou Cl et/ou groupes $CH_3$ et/ou CN,

$Z^1$ représente —$OCH_2$— ou —O—CO—,

$Z^2$ représente —CO—O—, —O—CO—, —$CH_2CH_2$—, —$CH_2O$—, —O—$CH_2$— ou une liaison simple et m vaut 0 ou 1,

avec cette précision que dans le cas où $Z^1$ = —O—CO—, $A^1$ représente un 1,4-phénylène non substitué et $A^2$ et $A^3$ représentent un trans-1,4-cyclohexylène ou un 1,4-phénylène non substitué.

19. Dérivés de pyrimidine selon la revendication 18, caractérisés en ce que $R^1$ et $R^2$ représentent chacun des groupes alcoyles ou alkoxy à chaîne droite ou ramifiée avec à chaque fois de 3 à 10 atomes de carbone.

20. Dérivés de pyrimidine selon la revendication 18 ou 19, caractérisés en ce que m vaut 0 et $A^2$ représente un trans-1,4-cyclohexylène ou un 1,4-phénylène non substitué.

21. Dérivés de pyrimidine selon au moins l'une des revendications 18 à 20, caractérisé en ce que $A^1$ est un 1,4-phénylène non substitué.

22. Composés à quatre noyaux de formule III

$$R^1-A^1-Z^3-A^{1'}-R^2 \qquad (III)$$

où

$R^1$ et $R^2$ représentent chacun un groupe alcoyle en $C_1$ à $C_{12}$, où également un ou plusieurs groupes $CH_2$ non voisins peuvent être remplacés par —O—, —CO—, —O—CO—, —O—COO—, —CO—O— et/ou —CH=CH—,

$A^1$ et $A^{1'}$ représentent chacun —Pyr—Phe—, —Phe—Pyr—, —Pyr—Cy— ou —Cy—Pyr—,

Pyr représente un pyrimidin-2,5-diyle,

Phe représente un 1,4-phénylène non substitué ou substitué par 1 ou 2 atomes de F et/ou Cl et/ou groupes $CH_3$ et/ou CN,

Cy représente un trans-1,4-cyclohexylène, et

$Z^3$ représente —CO—O— ou —$CH_2O$—.

23. Composés de formule III selon la revendication 22, caractérisés en ce que $R^1$ et $R^2$ représentent chacun des groupes alcoyles ou alkoxy à chaîne droite ou ramifiée avec à chaque fois de 3 à 10 atomes de carbone.

24. Composés selon au moins l'une des revendications 22 et 23, caractérisés en ce que $A^1$ et $A^{1'}$ représentent —Pyr—Phe— ou —Phe—Pyr—.

25. Composé selon au moins l'une des revendications 22 à 24, caractérisés par les formules partielles IIIa à IIId :

$R^1$—Pyr—Phe—COO—Phe—Pyr—$R^2$ IIIa

$R^1$—Pyr—Phe—COO—Pyr—Phe—$R^2$ IIIb

$R^1$—Pyr—Phe—$CH_2O$—Phe—Pyr—$R^2$ IIIc

$R^1$—Pyr—Phe—$CH_2O$—Pyr—Phe—$R^2$ IIId